(19) **Europäisches Patentamt / European Patent Office / Office européen des brevets**

(11) **EP 4 763 979 A1**

(12) **EUROPEAN PATENT APPLICATION**
published in accordance with Art. 153(4) EPC

(43) Date of publication:
**24.06.2026 Bulletin 2026/26**

(21) Application number: **24855769.6**

(22) Date of filing: **19.08.2024**

(51) International Patent Classification (IPC):
**C12N 5/10** (2006.01)    **C12N 15/113** (2010.01)

(52) Cooperative Patent Classification (CPC):
**C12N 5/10; C12N 15/113**

(86) International application number:
**PCT/CN2024/113033**

(87) International publication number:
**WO 2025/040046 (27.02.2025 Gazette 2025/09)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **18.08.2023 PCT/CN2023/113771**

(71) Applicant: **EdiGene Biotechnology, Inc.**
**Beijing 102206 (CN)**

(72) Inventors:
- **LIANG, Feifei**
  **Beijing 102206 (CN)**
- **ZHANG, Haixia**
  **Beijing 102206 (CN)**
- **YI, Yuting**
  **Beijing 102206 (CN)**
- **ZHANG, Yunyi**
  **Beijing 102206 (CN)**
- **SUN, Yu**
  **Beijing 102206 (CN)**
- **FU, Jianqiang**
  **Beijing 102206 (CN)**
- **YANG, Zhonghan**
  **Beijing 102206 (CN)**
- **WU, Lanlan**
  **Beijing 102206 (CN)**
- **LU, Zhiwei**
  **Beijing 102206 (CN)**
- **YUAN, Pengfei**
  **Beijing 102206 (CN)**

(74) Representative: **Lavoix**
**Bayerstraße 83**
**80335 München (DE)**

Remarks:
The complete document including Reference Table(s) and the Sequence Listing(s) can be downloaded from the EPO website

(54) **ENGINEERED T CELL HAVING PROLONGED IN VIVO HALF-LIFE AND PREPARATION METHOD THEREFOR**

(57) An engineered T cell having prolonged in vivo half-life and a preparation method therefor. The engineered T cell can reduce the killing of allogeneic T cells while avoiding the killing of NK cells.

**EP 4 763 979 A1**

**Description**

**TECHNICAL FIELD**

This application relates to modified immune cells, and more particularly to immune cells modified to reduce graft-versus-host disease (GvHD), activation-induced immune effector cell death (AICD), and host-versus-graft (HVG) effect in the host.

**BACKGROUND**

One of the biggest challenges in the development of universal CAR-T cell therapy (also known as U-CART, allogeneic chimeric antigen receptor T-cell therapy) is the persistence of U-CART cells. This is mainly due to three factors: First, through TCR-mediated attack, U-CART cells attack the patient's somatic cells, leading to graft-versus-host disease (GvHD); Second, CAR-T cells activated by target cells exhibit activation-induced cell death (AICD), caused by the simultaneous expression of FasL (CD95L) and Fas (CD95) by activated T cells. FasL is a cytotoxic effector molecule, and Fas can induce apoptosis of activated T cells through autocrine or paracrine FasL. Third, U-CART cells are attacked by the patient's T cells, leading to host-versus-graft (HVG) effect, which prevents U-CART cells from surviving long-term in the patient's body, thus greatly limiting their therapeutic effect. Currently, to address the persistence issue of U-CART cells, scientists generally choose to knock out the TCR gene in healthy donor T cells to prevent GvHD from occurring. At the same time, they intervene in the expression of B2M (which inhibits the expression of HLA-I molecules) on the surface of U-CART cells to prevent them from being recognized and attacked by patient T cells.

However, the above strategy can lead to another type of effector cell attacking U-CART cells that do not express HLA molecules. These effector cells are natural killer cells (NK cells). NK cells are effector immune cells that can recognize cells with low HLA expression. Many virus-infected cells and tumor cells downregulate the expression of HLA molecules in order to evade T cell recognition. U-CART cells whose HLA-related molecule expression is reduced due to intervention will also be cleared by the body through NK cell immunity via this mechanism.

Currently, there is no U-CART cell therapy that can simultaneously address AICD, heterologous T cell attack, and NK cell attack.

**SUMMARY OF THE INVENTION**

This application provides an engineered T cell with enhanced persistence and a preparation method thereof, which reduces GvHD, AICD and HVG without inducing NK cell killing, thus improving in vivo persistence and therapeutic efficacy.

Specifically, the first aspect of this application relates to an engineered T cell that is modified to eliminate or reduce the expression and/or function of an endogenous T cell receptor (TCR) protein and/or an SPPL3 protein. In some embodiments, the engineered T cells are $\gamma\delta$ T cells that are modified to eliminate or reduce the expression and/or function of the $\gamma$ subunit (TCR$\gamma$) and/or TCR$\delta$ subunit (TCR$\delta$) of their endogenous T cell receptor (TCR). In some embodiments, the engineered T cells are $\alpha\beta$ T cells that are modified to eliminate or reduce the expression and/or function of the $\alpha$ subunit (TCR$\alpha$) and/or $\beta$ subunit (TCR$\beta$) of the endogenous T cell receptor (TCR). In some embodiments, the modification comprises modification of the SPPL3 genomic region of chromosome 12 of the engineered T cell. In some embodiments, the modification comprises modification of the mRNA or pre-mRNA of the SPPL3 gene in the engineered T cells. In some embodiments, the engineered T cell is a human-derived T cell, and the modification comprises nonsense mutations in the SPPL3 genomic region or the mRNA (or pre-mRNA) of the SPPL3 gene of chromosome 12 of the engineered T cell. In some embodiments, the engineered T cells are human-derived T cells, and the modification occurs at any one or more nucleotide sites relative to any one or more exons of the SPPL3 gene, or between any two nucleotide sites.

Preferably, the modification occurs at any nucleotide site, or between any two nucleotide sites, corresponding to positions 120,903,845 to 120,904,358, 120,810,809 to 120,810,886, 120,791,469 to 120,791,557, 120,784,474 to 120,784,593, 120,783,674 to 120,783,752, 120,782,655 to 120,782,767, 120,768,953 to 120,769,059, 120,768,325 to 120,768,488, 120,767,394 to 120,767,593, 120,766,263 to 120,766,372, or 120,764,999 to 120,765,070 of chromosome 12 of the GRCh38.p14 reference genome. More preferably, the modification occurs at any nucleotide site, or between any two nucleotide sites, corresponding to positions 120,791,469 to 120,791,557, 120,783,674 to 120,783,752 or 120,784,474 to 120,784,593 of human chromosome 12. In some embodiments, the T cell is a human-derived T cell, and the modification comprises a modification of the TRAC genomic region of chromosome 14 of the engineered T cell. In some embodiments, the modification comprises modification of the mRNA or pre-mRNA of the TRAC gene in the engineered T cells. In some embodiments, the T cell is a human-derived T cell, and the modification comprises a nonsense mutation in the TRAC genome region or the TRAC gene of chromosome 14 of the engineered T cell. In some embodiments, the T cell is a human-derived T cell, and the modification occurs at any nucleotide site, or between any two nucleotide sites, corresponding to positions 23016448 to 23016490 of chromosome 14 of the GRCh38.p14 reference genome. In some embodiments, the modification results in a frameshift mutation. In some embodiments, the modification results in nonsense mutations.

In some embodiments, the T cells have normally expressed HMC class I proteins. In some embodiments, the T cells have normal expression of HLA-A, HLA-B, HLA-C, and B2M. In some embodiments, the T cells are human-derived T cells, and the expression and/or function of the B2M protein therein are not reduced.

In some embodiments, the engineered T cells further comprise or express engineered receptors. In some embodiments, the engineered receptor is one or more selected from the group consisting of: chimeric antigen receptor (CAR), engineered TCR, and T-cell antigen conjugate (TAC).

In some embodiments, the engineered T cells express a CAR, the CAR comprising i) an extracellular antigen-binding domain that specifically recognizes one or more target antigens or antigenic epitopes; ii) a transmembrane domain; and iii) an intracellular signaling domain. In some embodiments, the extracellular antigen-binding domain is one or more selected from the group consisting of: the extracellular domain of an antigen ligand, a single-domain antibody (sdAb), a single-chain Fv (scFv), and Fab. In some embodiments, the engineered T cells comprise one or more CARs, which have different extracellular antigen-binding domains. In some embodiments, the CAR has one or more different extracellular antigen-binding domains. In some embodiments, the different extracellular antigen-binding domains recognize different target antigens and/or different antigenic epitopes. In some embodiments, the extracellular antigen-binding domain binds to one or more proteins selected from: prostate stem cell antigen (PSCA), carcinoembryonic antigen (CEA), CAM5, CD123, thyroid-stimulating hormone receptor (TSHR); CD171; CS-1; C-type lectin-like molecule-1; ganglioside GD3; Tn antigen; CD19; CD20; CD22; CD30; CD70; CD123; CD 138; CD33; CD44; CD44v7/8; CD38; CD44v6; B7H3 (CD276), B7H6; KIT (CD117); interleukin-13 receptor subunit $\alpha$ (IL-13R$\alpha$); interleukin-11 receptor $\alpha$ (IL-11R$\alpha$); prostate-specific membrane antigen (PSMA); NY-ESO-1; HIV-1 Gag; MART-1; gp100; tyrosinase; mesothelin; EpCAM; protease serine 21 (PRSS21); vascular endothelial growth factor receptor; Lewis (Y) antigen; CD24; platelet-derived growth factor receptor $\beta$ (PDGFR-$\beta$); stage-specific embryonic antigen-4 (SSEA-4); cell surface-associated mucin 1 (MUC1), MUC6; epidermal growth factor receptor family and its mutants (EGFR, EGFR2, ERBB3, ERBB4, EGFRvIII); nerve cells adhesion molecule (NCAM); carbonic anhydrase IX (CAIX); LMP2; hepatic ligand type A receptor 2 (EphA2); fucosyl GM1; sialyl Lewis adhesion molecule (sLe); ganglioside GM3(aNeu5Ac(2-3)bDGalp(1-4)bDGlcp(1-1)Cer; TGS5; high molecular weight melanoma-associated antigen (HMWMAA); O-acetyl GD2 ganglioside (OAcGD2); folate receptor; tumor vascular endothelial marker 1 (TEM1/CD248); tumor vascular endothelial marker 7-associated (TEM7R); Claudin 6, Claudin 18.2, Claudin 18.1; ASGPR1; CDH16; 5T4; 8H9; $\alpha\nu\beta6$ integrin; B cell maturation antigen (BCMA); CA9; $\kappa$ light chain (kappa light chain); CSPG4; EGP2, EGP40; FAP; FAR; FBP; embryonic AchR; HLA-A1, HLA-A2, MAGEA1, MAGE3; KDR; MCSP; NKG2D ligand; PSC1; ROR1; Sp17; SURVIVIN; TAG72; TEM1; fibronectin; tenascins; carcinoembryonic variant of tumor necrosis region; G protein-coupled receptor class C group 5-member D (GPRC5D); X chromosome open reading frame 61 (CXORF61); CD97; CD179a; anaplastic lymphoma kinase (ALK); polysialic acid; placenta-specific 1 (PLAC1); the hexose moiety of Globo-H glycoceramide (GloboH); breast differentiation antigen (NY-BR-1); uroplakin 2 (UPK2); hepatitis A virus cell receptor 1 (HAVCR1); adrenaline receptor $\beta3$ (ADRB3); pannexin 3 (PANX3); G protein-coupled receptor 20 (GPR20); lymphocyte antigen 6 complex locus K9 (LY6K); olfactory receptor 51E2 (OR51E2); TCR$\gamma$ alternating reading frame protein (TARP); Wilms' blastoma protein (WT1); ETS translocation variant gene 6 (ETV6-AML); sperm protein 17 (SPA17); X antigen family member 1A (XAGE1); angiopoietin-binding cell surface receptor 2 (Tie2); melanoma-associated cancer testis antigen-1 (MAD-CT-1); melanoma-associated cancer testis antigen-2 (MAD-CT-2); Fos-associated antigen 1; p53 mutant; human telomerase reverse transcriptase (hTERT); sarcoma translocation breakpoint; melanoma inhibitor of apoptosis (ML-IAP); ERG (transmembrane protease serine 2 (TMPRSS2) ETS fusion gene); N-acetylglucosamine transferase V (NA17); pairing box protein Pax-3 (PAX3); androgen receptor; cyclin B1; V-myc myeloma virus oncogene neuroblastoma-derived homolog (MYCN); Ras homolog family member C (RhoC); cytochrome P4501B1 (CYP1B1); CCCTC binding factor (zinc finger protein)-like (BORIS); squamous cell carcinoma antigen 3 recognized by T cells (SART3); pairing box protein Pax-5 (PAX5); proacrosin-binding protein sp32 (OYTES1); lymphocyte-specific protein tyrosine kinase (LCK); A Kinase-anchored protein 4 (AKAP-4); synovial sarcoma, X-breakpoint 2 (SSX2); CD79a; CD79b; CD72; leukocyte-associated immunoglobulin-like receptor 1 (LAIR1); Fc fragment of IgA receptor (FCAR); leukocyte immunoglobulin-like receptor subfamily member 2 (LILRA2); CD300 molecular-like family member f (CD300LF); C-type lectin domain family 12 member A (CLEC12A); bone marrow stromal cell antigen 2 (BST2); mucin-like hormone receptor-like 2 compriseing EGF-like modules (EMR2); lymphocyte antigen 75 (LY75); phosphatidylinositol proteoglycan-3 (GPC3); Fc receptor-like 5 (FCRL5); immunoglobulin $\lambda$-like polypeptide 1 (IGLL1) and CD155. In some embodiments, the transmembrane domain comprises a transmembrane domain from any of the molecules selected from the group consisting of: TCR$\alpha$, TCR$\beta$, TCR$\gamma$, TCR$\delta$, CD3$\zeta$, CD3$\varepsilon$, CD3$\gamma$, CD3$\delta$, CD4, CD5, CD6, CD7, CD8$\alpha$, CD9, CD16, CD22, CD27, CD28, CD33, CD37, CD45, CD47, CD52, CD64, CD80, CD86, CD134, 4-1BB, CD152, CD154, CISH, and PD-1. In some embodiments, the intracellular signaling domain comprises a primary intracellular signaling domain comprising molecules selected from any of the group consisting of: CD3$\zeta$, CD3$\gamma$, CD3$\varepsilon$, CD3$\delta$, FcR$\gamma$, FcR$\beta$, CD5, CD22, CD79a, CD79b, CD66d, Fc$\gamma$RIIa, DAP10, and DAP12. In some embodiments, the intracellular signaling domain further comprises a co-stimulatory signaling domain comprising one or more co-stimulatory molecules selected from the group consisting of: CARD11, CD2, CD4, CD7, CD19, CD27, CD28, CD30, CD40, CD160, ICAM-1, OX40, 4-1BB, SELPLG, LIGHT, HVEM, B7-H3, ICOS, PD-1, SLAMF7, LFA-1, NKG2C, CDS, GITR, BAFFR, NKp80, IPO-3, SLAMF8, LTBR, LAT, GADS, SLP-76, PAG/Cbp, NKp44, NKp30, NKp46, NKG2D, CD83, SLAMF1, CTLA-4, LAG-3, PD-L2, PD-L1, DAP10, TRIM, ZAP70, a ligand that specifically binds to CD83, and any combination thereof. In some embodiments, the CAR further comprises a hinge domain located between the C-terminus of the extracellular antigen-binding domain and the N-terminus of the transmembrane domain. In some embodiments, the hinge domain is a hinge region of IgG, IgD, CD8$\alpha$, or CD28.

In some embodiments, the engineered T cells express a T cell antigen conjugate (TAC) comprising (i) an antigen-binding domain, (ii) a TCR-binding domain (e.g., scFv), and (iii) a co-receptor domain (e.g., a hinge, transmembrane, and/or cytosol region). In some embodiments, the TAC comprises: (a) an extracellular ligand-binding domain comprising an antigen-binding fragment (e.g., sdAb, scFv, Fab, DARPin) that specifically recognizes one or more target antigens (e.g., tumor antigens) or antigenic epitopes (e.g., tumor epitopes); (b) optionally a first adapter; (c) an extracellular TCR-binding domain (e.g., sdAb, scFv, Fab, DARPin) that specifically recognizes an extracellular domain of a TCR subunit (e.g., CD3$\epsilon$); (d) optionally a second adapter; (e) optionally an extracellular domain of a first TCR co-receptor (e.g., CD4, CD8) or a portion thereof; (f) a transmembrane domain comprising a transmembrane domain of a second TCR co-receptor (e.g., CD4, CD8); and (g) an optional intracellular signaling domain comprising an intracellular signaling domain of a third TCR co-receptor (e.g., CD4, CD8). In some embodiments, the engineered T cells comprise one or more TACs, wherein the one or more TACs have different antigen-binding domains or ligand-binding domains. In some embodiments, the TAC has one or more different antigen-binding domains or ligand-binding domains. In some embodiments, the different antigen-binding domains or ligand-binding domains recognize different target antigens and/or different antigenic epitopes. In some embodiments, the antigen-binding domain or the ligand-binding domain specifically recognizes one or more target antigens selected from the group consisting of: PSCA, CEACAM5, CD123, TSHR, CD171, CS-1, C-type lectin-like molecule-1, ganglioside GD3, Tn antigen, CD19, CD20, CD22, CD30, CD70, CD123, CD138, CD33, CD44, CD44v7/8, CD38, CD44v6, B7H3(CD276), B7H6, CD117, IL-13R$\alpha$, IL-11R$\alpha$, PSMA, NY-ESO-1, HIV-1 Gag, MART-1, gp100, tyrosinase, mesothelin, EpCAM, PRSS21, vascular endothelial growth factor receptor, Lewis (Y) antigen, CD24, PDGFR-$\beta$, SSEA-4, MUC1, MUC6, EGFR, EGFR2, ERBB3, ERBB4, EGFRvIII, NCAM, CAIX, LMP2, EphA2, fucosyl GM1, sLe, ganglioside GM3 (aNeu5Ac(2-3)bDGalp(1-4)bDGlcp(1-1)Cer, TGS5, HMWMAA, OAcGD2, folate receptor, CD248, TEM7R, Claudin 6, Claudin18.2, Claudin18.1, ASGPR1, CDH16, 5T4, 8H9, $\alpha\nu\beta$6 integrin, BCMA), CA9, $\kappa$ light chain, CSPG4, EGP2, EGP40, FAP, FAR, FBP, embryonic AchR, HLA-A1, HLA-A2, MAGEA1, MAGE3, KDR, MCSP, NKG2D ligand, PSC1, ROR1, Sp17, SURVIVIN, TAG72, TEM1, fibronectin, tenascins, carcinoembryonic variants in tumor necrosis region, GPRC5D, CXORF61, CD97, CD179a, ALK, polysialic acid, (PLAC1, GloboH, NY-BR-1, UPK2, HAVCR1, ADRB3, PANX3, GPR20, LY6K, OR51E2, TARP, WT1, ETV6-AML, SPA17, XAGE1, Tie2, MAD-CT-1, MAD-CT-2, Fos-associated antigen 1, p53 mutant, hTERT, sarcoma translocation breakpoint, ML-IAP, ERG, NA17, PAX3, androgen receptor, cyclin B1, MYCN, RhoC, CYP1B1, BORIS, SART3, (PAX5, OYTES1, (LCK, AKAP-4, SSX2, CD79a, CD79b, CD72, LAIR1, FCAR, LILRA2, CD300LF, CLEC12A, BST2, EMR2, LY75, GPC3, FCRL5, IGLL1, and CD155. In some embodiments, the TCR subunits are one or more selected from the group consisting of: CD3$\epsilon$, CD3$\delta$, CD3$\gamma$, TCR$\alpha$, TCR$\beta$, TCR$\gamma$, and TCR$\delta$; and wherein the first, second, and third TCR co-receptors are independently selected from any of the group consisting of: CD4, CD8, and CD28. In some embodiments, the first, second, and third TCR co-receptors are the same. In some embodiments, the first, second, and third TCR co-receptors are different. In some embodiments, the TAC further comprises a hinge domain located between the C-terminus of the extracellular ligand-binding domain and the N-terminus of the transmembrane domain. In some embodiments, the hinge domain is a hinge region of IgG, IgD, CD8$\alpha$, or CD28.

In some embodiments, the engineered T cells express engineered T cell receptors (TCRs) comprising: (a) an extracellular ligand-binding domain comprising an antigen-binding fragment (e.g., sdAb, scFv, Fab, DARPin) that specifically recognizes one or more target antigens (e.g., tumor antigens) or target epitopes (e.g., tumor epitopes); (b) optionally a first adapter; (c) optionally an extracellular domain of a first TCR subunit (e.g., C$\alpha$, C$\beta$, C$\delta$, C$\gamma$, CD3$\epsilon$) or a portion thereof; (d) a transmembrane domain of a second TCR subunit (e.g., TCR$\alpha$, TCR$\beta$); and (e) an intracellular signaling domain comprising an intracellular signaling domain of a third TCR subunit (e.g., TCR$\alpha$, TCR$\beta$); wherein the first, second, and third TCR subunits are independently selected from any one of the group consisting of: TCR$\alpha$, TCR$\beta$, TCR$\gamma$, TCR$\delta$, CD3$\epsilon$, CD3$\gamma$, CD3$\delta$, and CD3$\zeta$. In some embodiments, the first, second, and third TCR subunits are the same (e.g., all CD3$\epsilon$, all TCR$\alpha$, or all TCR$\beta$). In some embodiments, the first, second, and third TCR subunits are different. In some embodiments, the engineered T cell comprises one or more TCRs, and the one or more TCRs have different extracellular ligand binding domains. In some embodiments, the TCR has one or more different extracellular ligand binding domains. In some embodiments, the different extracellular ligand binding domains recognize different target antigens and/or different antigenic epitopes. In some embodiments, the antigen-binding domain or the ligand-binding domain specifically recognizes one or more target antigens selected from the following: PSCA, CEACAM5, CD123, TSHR, CD171, CS-1, C-type lectin-like molecule-1, ganglioside GD3, Tn antigen, CD19, CD20, CD22, CD30, CD70, CD123, CD138, CD33, CD44, CD44v7/8, CD38, CD44v6, B7H3(CD276), B7H6, CD117, IL-13Rα, IL-11Rα, PSMA, NY-ESO-1, HIV-1 Gag, MART-1, gp100, tyrosinase, mesothelin, EpCAM, PRSS21, vascular endothelial growth factor receptor, Lewis (Y) antigen, CD24, PDGFR-β, SSEA-4, MUC1, MUC6, EGFR, EGFR2, ERBB3, ERBB4, EGFRvIII, NCAM, CAIX, LMP2, EphA2, fucosyl GM1, sLe, ganglioside GM3(aNeu5Ac(2-3)bDGalp(1-4)bDGlcp(1-1)Cer, TGS5, HMWMAA, OAcGD2, folate receptor, CD248, TEM7R, Claudin 6, Claudin18.2, Claudin18.1, ASGPR1, CDH16, 5T4, 8H9, αvβ6 integrin, BCMA), CA9, κ light chain, CSPG4, EGP2, EGP40, FAP, FAR, FBP, embryonic AchR, HLA-A1, HLA-A2, MAGEA1, MAGE3, KDR, MCSP, NKG2D ligand, PSC1, ROR1, Sp17, SURVIVIN, TAG72, TEM1, fibronectin, tenascins, carcinoembryonic variants in tumor necrosis region, GPRC5D, CXORF61, CD97, CD179a, ALK, polysialic acid, (PLAC1, GloboH, NY-BR-1, UPK2, HAVCR1, ADRB3, PANX3, GPR20, LY6K, OR51E2, TARP, WT1, ETV6-AML, SPA17, XAGE1, Tie2, MAD-CT-1, MAD-CT-2, Fos-associated antigen 1, p53 mutant, hTERT, sarcoma translocation breakpoint, ML-IAP, ERG, NA17, PAX3, androgen receptor, cyclin B1, MYCN, RhoC, CYP1B1, BORIS, SART3, (PAX5, OYTES1, (LCK, AKAP-4, SSX2, CD79a, CD79b, CD72, LAIR1, FCAR, LILRA2, CD300LF, CLEC12A, BST2, EMR2, LY75, GPC3, FCRL5, IGLL1, and CD155. In some embodiments, the engineered TCR further comprises a hinge domain located between the C-terminus of the extracellular ligand-binding domain and the N-terminus of the transmembrane domain. In some embodiments, the hinge domain is derived from CD8α.

The second aspect of this application also provides a method for preparing the engineered T cells of the first aspect, wherein the modification comprises any one or more selected from the group consisting of: disruption or knockout of a coding gene, inhibition of coding gene transcription, disruption or clearance of mRNA, inhibition of coding gene expression, and inhibition of the protein. In some embodiments, the modification eliminates or reduces the expression of TCR or a functional fragment thereof and/or SPPL3 or a functional fragment thereof by RNA interference (RNAi). In some embodiments, the RNAi silences or inhibits the expression of TCR or a functional fragment thereof and/or SPPL3 or a functional fragment thereof by small interfering RNA (siRNA), short hairpin RNA (shRNA) or small RNA (miRNA). In some embodiments, the modification is achieved through physical mutagenesis, chemical mutagenesis, or transposition. In some embodiments, the modification is achieved by a PCR method. In some embodiments, the modification is mediated by one or more selected from the group consisting of: non-homologous end joining (NHEJ), homologous directed repair (HDR), zinc finger nuclease (ZFN), transcription activator-like effector nuclease (TALEN), CRISPR/Cas, and adenosine deaminase (ADAR). In some embodiments, the modification is gene editing, including, for example, base editing, PRIME editing, or enzyme cleavage of the target site. In some embodiments, the modification comprises one or more of insertion, deletion, and substitution. In some embodiments, the modification comprises one or more of translocation, point mutation, fragment deletion, and fragment addition. In some embodiments, the modification results in one or more selected from the group consisting of: frameshift mutation, loss-of-function mutation, dominant-negative mutation, missense mutation, and nonsense mutation. In some embodiments, the modification comprises CRISPR/Cas system-mediated gene editing or RNA editing using guide RNA (gRNA) targeting SPPL3 and/or TRAC. In some embodiments, the gRNA targeting SPPL3 comprises a guide sequence complementary to the SPPL3 genomic regions of human chromosome 12 (e.g., any exon region, any intron region, any splice site therein) or to the mRNA or pre-mRNA sequences corresponding to the genomic regions. Preferably, the gRNA targeting SPPL3 comprises a guide sequence that is complementary to the SPPL3 genomic regions at positions 120,903,845 to 120,904,358, 120,810,809 to 120,810,886, 120,791,469 to 120,791,557, 120,784,474 to 120,784,593, 120,783,674 to 120,783,752, 120,782,655 to 120,782,767, 120,768,953 to 120,769,059, 120,768,325 to 120,768,488, 120,767,394 to 120,767,593, 120,766,263to 120,766,372 or 120,764,999 to 120,765,070 of human chromosome 12, or to the mRNA or pre-mRNA sequences corresponding to the genomic regions. More preferably, the gRNA targeting SPPL3 comprises a guide sequence complementary to the SPPL3 genomic regions at positions 120,791,469 to 120,791,557, 120,783,674 to 120,783,752, or 120,784,474 to 120,784,593 of human chromosome 12, or the mRNA or pre-mRNA sequences corresponding to the genomic regions. In some embodiments, the gRNA targeting TRAC comprises a guide sequence complementary to the TRAC genomic region at positions 23016448 to 23016490 of human chromosome 14, or the mRNA or pre-mRNA sequences corresponding to the genomic region. In some embodiments, the CRISPR/Cas system is a type II CRISPR/Cas system, wherein the gRNA, in addition to the guide sequence, comprise tracrRNA or tracr-chaperone sequences. In some embodiments, the CRISPR/Cas system is a type II CRISPR/Cas system, wherein the gRNA is a single-guide RNA (sgRNA). In some embodiments, the CRISPR/Cas system is a CRISPR/Cas9 system or a CRISPR/Cas12 system. In some embodiments, the guide sequence complementary to the SPPL3 genomic region is one or more selected from the group consisting of: SEQ ID NO:68, SEQ ID NO:145, SEQ ID NO:150, SEQ ID NO:154, SEQ ID NO:155, SEQ ID NO:159, SEQ ID NO:161, SEQ ID NO:165, SEQ ID NO:172, SEQ ID NO:176, SEQ ID NO:196, SEQ ID NO:203, SEQ ID NO:204, SEQ ID NO:209, SEQ ID NO:212, SEQ ID NO:241, SEQ ID NO:250, SEQ ID NO:261, SEQ ID NO:269, SEQ ID NO:270, SEQ ID NO:271, SEQ ID NO:272, SEQ ID NO:273, SEQ ID NO:274, SEQ ID NO:275, SEQ ID NO:276, SEQ ID NO:277, SEQ ID NO:278, SEQ ID NO:279, SEQ ID NO:280, SEQ ID NO:281, SEQ ID NO:282, SEQ ID NO:283, SEQ ID NO:284, SEQ ID NO:285, SEQ ID NO:286, SEQ ID NO:287, SEQ ID NO:288, SEQ ID NO:289, SEQ ID NO:290, SEQ ID NO:291, SEQ ID NO:292, SEQ ID NO:293, SEQ ID NO:294, SEQ ID NO:295, SEQ ID NO:297, SEQ ID NO:300, SEQ ID NO:305, SEQ ID NO:312 and SEQ ID NO:315.

In some embodiments, the guide sequence complementary to the SPPL3 genomic region is SEQ ID NO:150, SEQ ID NO:154, SEQ ID NO:155, SEQ ID NO:159, SEQ ID NO:161, SEQ ID NO:269, SEQ ID NO:270, SEQ ID NO:271, SEQ ID NO:272, SEQ ID NO:273, SEQ ID NO:274, SEQ ID NO:275, SEQ ID NO:276, SEQ ID NO:277, SEQ ID NO:278, SEQ ID NO:279, SEQ ID NO:280, SEQ ID NO:281, SEQ ID NO:282, SEQ ID NO:283, SEQ ID NO:284, SEQ ID NO:285, SEQ ID NO:286, SEQ ID NO:287, SEQ ID NO:288, SEQ ID NO:289, SEQ ID NO:290, SEQ ID NO:291, SEQ ID NO:292, SEQ ID NO:293 or SEQ ID NO:294. In some embodiments, the guide sequence complementary to the SPPL3 genomic region is SEQ ID NO:155 or SEQ ID NO:261. In some embodiments, the guide sequence complementary to the TRAC genome is SEQ ID NO:1, SEQ ID NO:2, SEQ ID NO:257, SEQ ID NO:258, SEQ ID NO:259 and/or SEQ ID NO:260. In some embodiments, the gRNA is chemically modified. In some embodiments, the chemical modification comprises 2'-O-methylation of the ribose of the nucleotide or 3'-thiophosphate bond modification between nucleotides, or both. In some embodiments, the modification is a 2'-O-methylation modification on the ribose of the first three nucleotides at the 5' end, a 2'-O-methylation modification on the ribose of the last three nucleotides at the 3' end, an internucleotide 3' thiophosphorylation modification on the first three nucleotides at the 5' end, and an internucleotide 3' thiophosphorylation modification on the last three nucleotides at the 3' end.

A third aspect of this application also provides a method for prolonging the in vivo half-life of engineered cells, comprising modifying the engineered cells to eliminate or reduce the expression and/or function of SPPL3 protein or a functional fragment thereof. In some embodiments, the modification comprises one or more of insertion, deletion, and substitution. In some embodiments, the modification comprises one or more of translocation, point mutation, fragment deletion, and fragment addition. In some embodiments, the modification results in one or more selected from the group consisting of: frameshift mutation, loss-of-function mutation, dominant-negative mutation, missense mutation, and nonsense mutation. In some embodiments, the modification is located at the SPPL3 genomic site. In some embodiments, the modification occurs in the mRNA transcribed from the SPPL3 gene. In some embodiments, the modification occurs in the exons. In some embodiments, the modification occurs in the intron. In some embodiments, the modification does not alter any genomic site of SPPL3 but downregulates mRNA by interfering with SPPL3 transcription. In some embodiments, the modification does not interfere with any genomic site or mRNA level of SPPL3, but interferes with the process of mRNA translation into SPPL3 protein, thereby leading to downregulation of SPPL3 protein levels. In some embodiments, the modification causes a mutation in the SPPL3 genomic regions of human chromosome 12 or the mRNA or pre-mRNA sequences corresponding to the genomic regions. In some embodiments, the modification causes nonsense mutations in the SPPL3 genomic regions of human chromosome 12 or the mRNA or pre-mRNA sequences corresponding to the genomic regions. In some embodiments, the engineered cells have or express normal levels of MHC-I protein. In some embodiments, the engineered cells have normal expression of HLA-A, HLA-B, HLA-C, and B2M. In some embodiments, the normal level of MHC-I protein does not cause NK cells to kill the engineered cells. In some embodiments, the engineered cells are immune cells or their precursor cells. In some embodiments, the immune cells are one or more selected from the group consisting of: T cells, B cells, natural killer (NK) cells, macrophages, and DC cells. In some embodiments, the immune cells are αβT cells, and the expression and/or function of endogenous TCRα and/or TCRβ or a functional fragment thereof in the αβT cells is eliminated or reduced. In some embodiments, the engineered T cells are γδT cells that are modified to eliminate or reduce the expression and/or function of their endogenous TCRγ and/or TCRδ. In some embodiments, the engineered cells further comprise or express engineered receptors. In some embodiments, the engineered receptor is one or more selected from the group consisting of chimeric antigen receptors (CARs), engineered TCRs, and T-cell antigen conjugates (TACs).

The fourth aspect of this application also provides a guide RNA (gRNA) targeting SPPL3, comprising a guide sequence selected from any of the following: SEQ ID NO:68, SEQ ID NO:145, SEQ ID NO:150, SEQ ID NO:154, SEQ ID NO:155, SEQ ID NO:159, SEQ ID NO:161, SEQ ID NO:165, SEQ ID NO:172, SEQ ID NO:176, SEQ ID NO:196, SEQ ID NO:203, SEQ ID NO:204, SEQ ID NO:209, SEQ ID NO:212, SEQ ID NO:241, SEQ ID NO:250, SEQ ID NO:261, SEQ ID NO:269, SEQ ID NO:270, SEQ ID NO:271, SEQ ID NO:272, SEQ ID NO:273, SEQ ID NO:274, SEQ ID NO:275, SEQ ID NO:276, SEQ ID NO:277, SEQ ID NO:278, SEQ ID NO:279, SEQ ID NO:280, SEQ ID NO:281, SEQ ID NO:282, SEQ ID NO:283, SEQ ID NO:284, SEQ ID NO:285, SEQ ID NO:286, SEQ ID NO:287, SEQ ID NO:288, SEQ ID NO:289, SEQ ID NO:290, SEQ ID NO:291, SEQ ID NO:292, SEQ ID NO:293, SEQ ID NO:294, SEQ ID NO:295, SEQ ID NO:297, SEQ ID NO:300, SEQ ID NO:305, SEQ ID NO:312 and SEQ ID NO:315, preferably, comprising a guide sequence selected from any of the following: SEQ ID NO:150, SEQ ID NO:154, SEQ ID NO:155, SEQ ID NO:159, SEQ ID NO:161, SEQ ID NO:269, SEQ ID NO:270, SEQ ID NO:271, SEQ ID NO:272, SEQ ID NO:273, SEQ ID NO:274, SEQ ID NO:275, SEQ ID NO:276, SEQ ID NO:277, SEQ ID NO:278, SEQ ID NO:279, SEQ ID NO:280, SEQ ID NO:281, SEQ ID NO:282, SEQ ID NO:283, SEQ ID NO:284, SEQ ID NO:285, SEQ ID NO:286, SEQ ID NO:287, SEQ ID NO:288, SEQ ID NO:289, SEQ ID NO:290, SEQ ID NO:291, SEQ ID NO:292, SEQ ID NO NO:293 and SEQ ID NO:294.

In some embodiments, the gRNA participates in the formation of the type II CRISPR/Cas complex. In some embodiments, the gRNA participates in the formation of the CRISPR/Cas complex, wherein the Cas enzyme is Cas9 or Cas12. In some embodiments, the CRISPR/Cas system is a type II CRISPR/Cas system, wherein the gRNA, in addition to the guide sequence, comprises tracrRNA or tracr-chaperone sequences. In some embodiments, the CRISPR/Cas system is a type II CRISPR/Cas system, wherein the gRNA is sgRNA. In some embodiments, the gRNA is chemically modified. In some embodiments, the chemical modification comprises 2'-O-methylation of the ribose of the nucleotide or 3'-thiophosphate bond modification between nucleotides, or both. In some embodiments, the modification is a 2'-O-methylation modification on the ribose of the first three nucleotides at the 5' end, a 2'-O-methylation modification on the ribose of the last three nucleotides at the 3' end, an internucleotide 3' thiophosphorylation modification on the first three nucleotides at the 5' end, and an internucleotide 3' thiophosphorylation modification on the last three nucleotides at the 3' end.

The fifth aspect of this application provides a nucleic acid composition comprising the gRNA of the fourth aspect above and gRNA targeting the T cell receptor gene. In some embodiments, the T-cell receptor gene is the TRAC gene. In some embodiments, the gRNA targeting the T cell receptor gene comprises a guide sequence complementary to the TRAC genomic region at positions 23016448 to 23016490 of chromosome 14. In some embodiments, the guide sequence comprises a polynucleotide sequence as shown in SEQ ID NO:1, SEQ ID NO:2, SEQ ID NO:257, SEQ ID NO:258, SEQ ID NO:259 or SEQ ID NO:260.

In some embodiments, the nucleic acid composition comprises at least two gRNAs, one of which comprises a guide sequence as shown in any one of: SEQ ID NO:68, SEQ ID NO:145, SEQ ID NO:150, SEQ ID NO:154, SEQ ID NO:155, SEQ ID NO:159, SEQ ID NO:161, SEQ ID NO:165, SEQ ID NO:172, SEQ ID NO:176, SEQ ID NO:196, SEQ ID NO:203, SEQ ID NO:204, SEQ ID NO:209, SEQ ID NO:212, SEQ ID NO:241, SEQ ID NO:250, SEQ ID NO:261, SEQ ID NO:269, SEQ ID NO:270, SEQ ID NO:271, SEQ ID NO:272, SEQ ID NO:273, SEQ ID NO:274, SEQ ID NO:275, SEQ ID NO:276, SEQ ID NO:277, SEQ ID NO:278, SEQ ID NO:279, SEQ ID NO:280, SEQ ID NO:281, SEQ ID NO:282, SEQ ID NO:283, SEQ ID NO:284, SEQ ID NO:285, SEQ ID NO:286, SEQ ID NO:287, SEQ ID NO:288, SEQ ID NO:289, SEQ ID NO:290, SEQ ID NO:291, SEQ ID NO:292, SEQ ID NO:293, SEQ ID NO:294, SEQ ID NO:295, SEQ ID NO:297, SEQ ID NO:300, SEQ ID NO:305, SEQ ID NO:312, and SEQ ID NO:315; preferably, it comprises a guide sequence as shown in any one of: SEQ ID NO:150, SEQ ID NO:154, SEQ ID NO:155, SEQ ID NO:159, SEQ ID NO:161, SEQ ID NO:269, SEQ ID NO:270, SEQ ID NO:271, SEQ ID NO:272, SEQ ID NO:273, SEQ ID NO:274, SEQ ID NO:275, SEQ ID NO:276, SEQ ID NO:277, SEQ ID NO:278, SEQ ID NO:279, SEQ ID NO:280, SEQ ID NO:281, SEQ ID NO:282, SEQ ID NO:283, SEQ ID NO:284, SEQ ID NO:285, SEQ ID NO:286, SEQ ID NO:287, SEQ ID NO:288, SEQ ID NO:289, SEQ ID NO:290, SEQ ID NO:291, SEQ ID NO:292, SEQ ID NO:293 and SEQ ID NO:294; wherein the other gRNA targets the T cell receptor gene. In some embodiments, the nucleic acid composition comprises at least two gRNAs, one of which comprises a guide sequence as shown in SEQ ID NO:155 or SEQ ID NO:261, and the other of which is a gRNA that targets the T cell receptor gene. In some embodiments, the nucleic acid composition comprises at least two gRNAs, one of which comprises a guide sequence as shown in SEQ ID NO:155 or SEQ ID NO:261, and the other comprises a guide sequence complementary to the TRAC genomic region at positions 23016448 to 23016490 of chromosome 14. In some embodiments, the nucleic acid composition comprises at least two gRNAs, one of which comprises a guide sequence as shown in SEQ ID NO:155 or SEQ ID NO:261, and the other comprises a guide sequence as shown in SEQ ID NO:2 or SEQ ID NO:257.

## BRIEF DESCRIPTION OF THE DRAWINGS

Figures 1A and 1B show the proliferation and viability of U-CART constructed by the CRISPR/Cas9 system on days 0-9 before purification. The sgRNA1-sgRNA17-SP3-3 represents the combination of SEQ ID NO:1 targeting TRAC with SEQ ID NO:68, SEQ ID NO:145, SEQ ID NO:150, SEQ ID NO:154, SEQ ID NO:155, SEQ ID NO:159, SEQ ID NO:161, SEQ ID NO:165, SEQ ID NO:172, SEQ ID NO:176, SEQ ID NO:196, SEQ ID NO:203, SEQ ID NO:204, SEQ ID NO:209, SEQ ID NO:212, SEQ ID NO:241, and SEQ ID NO:250 targeting SPPL3 to double knock out both TRAC and SPLL3.

Figure 2A shows the gene knockout efficiency of the TCR of U-CART constructed by the CRISPR/Cas9 system on day 9 before purification.

Figure 2B shows the gene knockout efficiency of SPPL3 of U-CART constructed by the CRISPR/Cas9 system on day 9 before purification.

Figure 2C shows the percentage of CAR+ cells of the U-CART constructed by the CRISPR/Cas9 system on day 9 before purification.

Figure 2D shows the viability of U-CART constructed by the CRISPR/Cas9 system on day 9 before purification.

Figure 2E shows the cell number of U-CART constructed by the CRISPR/Cas9 system on day 9 before purification.

Figures 3A and 3B show the proliferation and viability of U-CART constructed by the CRISPR/Cas12 system on days 0-9 before purification. UCART-TCR-sg1, UCART-TCR-sg2, UCART-TCR-sg3, and UCART-TCR-sg4 represent the sequences targeting TRAC (SEQ ID NO:257, SEQ ID NO:258, SEQ ID NO:259, and SEQ ID NO:260), respectively; SPPL3-sg1, SPPL3-sg2, SPPL3-sg3, and SPPL3-sg4 represent the sequences targeting SPPL3 (SEQ ID NO:290, SEQ ID NO:269, SEQ ID NO:261, and SEQ ID NO:270), respectively. These sequences are combined to double knock out both TRAC and SPPL3.

Figure 4A shows the gene knockout efficiency of the TCRs of U-CART constructed by the CRISPR/Cas12 system on day 9 before purification.

Figure 4B shows the gene knockout efficiency of SPPL3 of U-CART constructed by the CRISPR/Cas 12 system on day 9 before purification.

Figure 4C shows the percentage of CAR+ cells of U-CART constructed by the CRISPR/Cas12 system on day 9 before purification.

Figure 4D shows the viability of U-CART constructed by the CRISPR/Cas12 system on day 9 before purification.

Figure 4E shows the cell number of U-CART constructed by the CRISPR/Cas12 system on day 9 before purification.

Figure 5A shows the change in TCR knockout efficiency of U-CART constructed by the CRISPR/Cas9 system before and after purification.

Figure 5B shows the change in the viability of U-CART constructed by the CRISPR/Cas9 system before and after purification.

Figures 6A and 6B show a comparison of the proliferation fold (2A) and viability (2B) of U-CART and CAR-T cells constructed by the CRISPR/Cas9 system with those of T cells.

Figure 6C shows the change in long-term viability of U-CART constructed by the CRISPR/Cas9 system.

Figure 7A shows the changes in TCR knockout efficiency of U-CART constructed by the CRISPR/Cas12 system before and after purification.

Figure 7B shows the change in the viability of U-CART constructed by the CRISPR/Cas12 system before and after purification.

Figures 8A and 8B show a comparison of the proliferation fold (2A) and viability (2B) of U-CART and CAR-T cells constructed by the CRISPR/Cas9 system with those of T cells.

Figure 8C shows the change in long-term viability of U-CART constructed by the CRISPR/Cas12 system.

Figure 9 shows the resistance of T cells from two donors to the killing effect of allogeneic T cells after different modifications.

Figure 10 shows the killing effect of NK cells on T cells from the same donor with different modifications.

Figures 11A and 11B show the killing effect of PBMC cells on T cells with different modifications.

Figure 12 shows the FasL-mediated killing effect on T cells with different modifications.

Figure 13 shows the killing effect of T cells with different modifications on target cells in vitro, wherein U-CART cells were constructed based on the CRISPR/Cas9 system.

Figure 14 shows the killing effect of T cells with different modifications on target cells in vitro, wherein U-CART cells were constructed based on the CRISPR/Cas12 system.

Figure 15 shows the proliferation of CAR+ cells when T cells with different modifications are co-incubated with target cells in vitro.

Figure 16 shows the results of in vivo imaging of mice.

Figure 17 shows the killing effect of T cells with different modifications on target cells in immunodeficient mice.

## DETAIL DESCRIPTION OF THE INVENTION

The inventors of this application have creatively provided a method for reducing the HVG effect of allogeneic T cells on engineered cells, which is different from knocking out MHC-I or inhibiting MHC-I expression and/or function. This method reduces HVG without increasing the killing effect of NK cells on the engineered cells. Specifically, this application also provides engineered T cells modified by the method, a method for preparing said engineered T cells, and gRNA and combinations thereof used in the method.

Definition

This application will be described in conjunction with specific embodiments and with reference to certain accompanying drawings, but is not limited thereto. Any reference numerals in the claims should not be construed as limiting their scope. In the accompanying drawings, for illustrative purposes, the dimensions of some elements may be exaggerated and not drawn to scale. Unless otherwise defined, all technical and scientific terms used herein have the same meaning as commonly understood by one of ordinary skill in the art. In case of any conflict, this document (including definitions) shall prevail. Preferred methods and materials are described below, although similar or equivalent methods and materials may be used in the practice or testing of this application. All publications, patent applications, patents and other references mentioned herein are incorporated herein by reference in their entirety. The materials, methods, and embodiments disclosed herein are illustrative only and are not intended to be limiting.

As used herein, "SPPL3" refers to signal peptidase-like 3, also known as IMP2, PSH1, or PSL4, which is a multi-transmembrane protein located in the Golgi-associated vesicle membrane, plasma membrane, and rough endoplasmic reticulum. SPPL3 possesses aspartate endopeptidase activity, intramembrane cleavage, and protein homodimerization activity. Many of its substrates are located in the Golgi apparatus and it is involved in N- and O-linked sugar modification and the biosynthesis of glycosaminoglycans. SPPL3 is crucial for the cleavage and extracellular release of the luminal domains of glycosyltransferases and glycosidases. The loss of sugar-modifying enzymes can impair their activity in the Golgi apparatus. Increased SPPL3 expression is associated with hypoglycosylation of many secretory and membrane proteins; decreased SPPL3 expression is associated with hyperglycosylated proteins. In the absence of SPPL3, researchers noted the upregulation of neolacto-series glycosphingolipids (GSLs) on the cell surface, which in turn hindered the interaction of antibodies and receptors with HLA class I (HLA-I) glycoproteins and reduced CD8+ T cell activation. SPPL3 also has non-proteolytic functions, including interacting with matrix-interacting molecules 1 (STIM1) and Orai1 to enhance TCR signaling, thereby greatly inducing calcium influx and NFAT activation, which are crucial for lymphocyte signaling. An example SPPL3 is a human SPPL3, such as the SPPL3 with gene ID 121665 in the NCBI database. In some embodiments, the gene sequence of human SPPL3 is shown as positions 120762510 to 120904358 of NC_000012.12, which is positions 120762510 to 120904358 of chromosome 12 of the GRCh38.p14 reference genome.

The "CRISPR system" or "CRISPR/Cas system" refers to transcripts and other elements involved in the expression of CRISPR-related ("Cas") genes and/or guiding their activity. For example, a CRISPR/Cas system may comprise a sequence encoding the Cas gene, a tracr (trans-activating CRISPR) sequence (e.g., tracrRNA or the active portion of tracrRNA), a tracr-chaperone sequence (e.g., in endogenous CRISPR systems, it may comprise a "direct repeat" and a partially direct repeat processed by tracrRNA), a guide sequence (also referred to as a "spacer" in endogenous CRISPR systems), and other sequences and transcripts derived from CRISPR loci.

In the context of CRISPR complex formation, the "target sequence" refers to the sequence to which the guide sequence is designed to be complementary, and hybridization between the target sequence and the guide sequence promotes the formation of the CRISPR complex. Perfect complementarity is not necessarily required if sufficient complementarity exists to induce hybridization and promote the formation of the CRISPR complex. The target sequence can comprise any polynucleotide, such as DNA or RNA polynucleotides. CRISPR complexes may comprise a guide sequence that hybridizes to a target sequence and complexes with one or more Cas proteins.

The term "guide sequence" refers to a continuous nucleotide sequence in guide RNA that is partially or completely complementary to the target sequence in the target polynucleotide and can hybridize with the target sequence through base pairing facilitated by the Cas protein. In the CRISPR/Cas9 system, the target sequence is adjacent to the PAM site. The PAM sequence and its complementary sequence on the other strand together constitute the PAM site.

The term "guide RNA" is used interchangeably with gRNA herein and refers to nucleic acid-based molecules, including but not limited to those capable of forming a protein-RNA complex with Cas proteins and comprising a sequence (e.g., a guide sequence or spacer) that is fully complementary to the target sequence, to hybridize with the target sequence and guide the Cas protein-RNA complex to bind specifically to the target sequence. In some embodiments, gRNA comprises or is crRNA. In some embodiments, the gRNA comprises two RNA strands, wherein the spacer sequence and the direct repeat (DR) sequence are in different RNA strands, for example, a crRNA strand and a tracrRNA strand. In some embodiments, gRNA is a single RNA strand, such as sgRNA.

The terms "single guide RNA," "synthetic guide RNA," and "sgRNA" are used interchangeably and refer to a polynucleotide sequence comprising a guide sequence and any other sequences that are necessary for the function of the sgRNA and/or necessary for the sgRNA to interact with one or more Cas proteins to form a CRISPR complex. In some embodiments, the sgRNA comprises a guide sequence fused to a second sequence, the second sequence comprising a tracr sequence derived from tracrRNA and a tracr chaperone sequence derived from crRNA. The tracr sequence can comprise all or part of the tracrRNA sequence from the naturally occurring CRISPR/Cas system. The term "guide sequence" is the nucleotide sequence in guide RNA that specifies the target site, and it can be used interchangeably with the terms "guide" or "spacer". The term "tracr chaperone sequence" can also be used interchangeably with the term "direct duplication". As used herein, "sgRNA$^{iBAR}$" refers to a single-guide RNA with an iBAR sequence.

As used in this application, "universal CAR-T" or "U-CART" cells refer to CAR-T (or CART) cells that have reduced GvHD and/or HVG effects relative to allogeneic individuals. The reduction can be achieved, for example, by eliminating or reducing TCR protein expression and/or function. In some embodiments, the U-CART is one type of engineered T cell provided in this application. The term "CAR-T" or "CART" cell refers to a T cell that expresses and/or comprises a chimeric antigen receptor (CAR) on its cell membrane.

As used herein, the term "wildtype" is a term understood by those skilled in the art and refers to the typical form of an organism, strain, gene, or trait as it appears in nature, distinct from mutant or variant forms.

As used herein, the term "variant" should be understood as a manifestation that exhibits characteristics that deviate from the naturally occurring pattern.

"Complementarity" refers to the ability of a nucleic acid to form hydrogen bonds with another nucleic acid sequence through traditional Watson-Crick base pairing or other non-traditional types. Percentage complementarity indicates the percentage of residues in a nucleic acid molecule that can form hydrogen bonds with a second nucleic acid sequence (e.g., Watson-Crick base pairing) (e.g., 50%, 60%, 70%, 80%, 90%, and 100% complementarity for 5, 6, 7, 8, 9, and 10 out of 10). "Complete complementarity" means that all consecutive residues in the nucleic acid sequence form hydrogen bonds with the same number of consecutive residues in the second nucleic acid sequence. As used herein, "basic complementarity" refers to a degree of complementarity of at least 60%, 65%, 70%, 75%, 80%, 85%, 90%, 95%, 97%, 98%, 99%, or 100% in 8, 9, 10, 11, 12, 13, 14, 15, 16, 17, 18, 19, 20, 21, 22, 23, 24, 25, 30, 35, 40, 45, 50 or more nucleotide regions, or to two nucleic acids that hybridize under strict conditions.

As used herein, the "strict condition" for hybridization refers to the condition that nucleic acids complementary to the target sequence hybridize primarily with the target sequence and substantially do not hybridize with non-target sequences. Strict conditions are typically sequence-dependent and vary depending on numerous factors. Generally, the longer the sequence, the higher the temperature at which it specifically hybridizes with its target sequence. A detailed description of non-restrictive examples of strict conditions can be found in Tijssen (1993), Laboratory Techniques In Biochemistry And Molecular Biology - Hybridization With Nucleic Acid Probes Part 1, Second Chapter "Overview of principles of hybridization and the strategy of nucleic acid probe assay", Elsevier, N.Y.

"Hybridization" refers to the reaction in which one or more polynucleotides form a complex that is stable through hydrogen bonds between the bases of nucleotide residues. Hydrogen bonds can occur through Watson-Crick base pairing, Hoogstein binding, or any other sequence-specific mechanism. The complex may comprise a double strand forming a double helix, three or more strands forming a multi-stranded complex, a single self-hybridizing strand, or any combination thereof. Hybridization reactions can constitute steps in a broader process, such as the initiation of PCR or the cleavage of polynucleotides by enzymes. A sequence that can hybridize with a given sequence is called the "complementary sequence" of the given sequence.

As used herein, "construction" refers to a nucleic acid molecule (e.g., DNA or RNA) or a vector capable of delivering such nucleic acid molecules. For example, when used in the context of gRNA or sgRNA, construct refers to a gRNA or sgRNA molecule, a nucleic acid molecule encoding gRNA or sgRNA (e.g., isolated DNA or viral vector), or a vector capable of delivering a nucleic acid molecule encoding gRNA or sgRNA, such as a lentivirus carrying a nucleic acid molecule encoding gRNA or sgRNA. When used in the context of proteins, construct refers to a nucleic acid molecule that comprises a nucleotide sequence that can be transcribed into RNA or expressed as a protein. The construct may comprise essential regulatory elements that are operatively linked to a nucleotide sequence, which allow the transcription or expression of that nucleotide sequence when the construct is present in a host cell. As used herein, "operatively linked" means that gene expression is under the control of regulatory elements (e.g., promoters) that are spatially linked to it. Regulatory elements can be located at the 5' (upstream) or 3' (downstream) of the gene they control. The distance between a regulatory element (e.g., a promoter) and a gene can be approximately the same as the distance between the regulatory element (e.g., a promoter) and the gene it naturally controls, and the regulatory element is derived from that gene. As is known in the art, the change in distance can be adapted without losing the function of the regulating element (e.g., the promoter). The term "vector" is used to describe a nucleic acid molecule that can be engineered to comprise one or more polynucleotides of a clone which can be amplified in a host cell. The vector includes, but is not limited to, single-stranded, double-stranded or partially double-stranded nucleic acid molecules; nucleic acid molecules comprising one or more free ends or without free ends (e.g., circular); nucleic acid molecules comprising DNA, RNA or both; and other types of polynucleotides known in the art. One type of vector is a "plasmid," which is a circular double-stranded DNA loop into which additional DNA fragments can be inserted, such as through standard molecular cloning techniques. Some vectors are able to replicate autonomously in the host cells in which they are introduced (e.g., bacterial vectors with bacterial origins of replication and free mammalian vectors). Other vectors (e.g., non-free mammalian vectors) are integrated into the host cell's genome after being introduced into the host cell, thereby replicating along with the host genome. In addition, some vectors can guide the expression of those genes that they are operatively linked to. This type of carrier is referred to as an "expression carrier" herein. The recombinant expression vector may comprise the nucleic acid of this application in a form suitable for expression in a host cell, which means that the recombinant expression vector comprises one or more regulatory elements that can be selected for expression based on the host cell, i.e., operatively linked to the nucleic acid sequence to be expressed.

"Host cell" refers to a cell that can be or already is a vector or a receptor for isolated polynucleotides. The host cell can be a prokaryotic cell or a eukaryotic cell. In some embodiments, the host cell is a eukaryotic cell that can be cultured in vitro and modified using the methods described herein. The term "cell" comprises primary test cells and their progeny.

As used herein, the term "autologous" means any material from the same individual that will later be reintroduced into that individual.

"Allogeneic" refers to grafts from different individuals of the same species. "Allogeneic T cells" refer to T cells derived from a donor that have a tissue human leukocyte antigen (HLA) type that matches that of the recipient. Typically, matching is based on the variability of three or more loci in the HLA gene, with a preference for perfect matches at these loci. In certain cases, allogeneic transplant donors may be related (usually closely HLA-matched siblings), genetically identical (the patient's monozygotic twins), or unrelated (donors who are not related by blood but are found to be very closely HLA-matched). HLA genes are divided into two types (type I and type II). Generally speaking, mismatches of type I genes (i.e., HLA-A, HLA-B, or HLA-C) increase the risk of transplant rejection. Mismatches in HLA type II genes (i.e., HLA-DR or HLA-DQB1) increase the risk of graft-versus-host disease (GvHD).

The term "donor subject" or "donor" herein refers to a subject whose cells are obtained for further in vitro engineering. The donor subject can be a patient to be treated with the cell population generated by the method described herein (i.e., an autologous donor), or an individual who donates a blood sample (e.g., a lymphocyte sample) and, after the cell population is generated by the method described herein, the cell population will be used to treat different individuals or patients (i.e., an allogeneic donor). Subjects who receive modified cells (such as those described herein or modified immune cells prepared by this method) may be referred to as "recipients" or "recipient subjects".

As used herein, a cell's "phenotype" refers to the observable characteristics or traits of a cell, such as its morphology, development (e.g., growth, proliferation, differentiation, or death), homeostasis, biochemical or physiological properties, phenology, or behavior. Phenotypes may arise from gene expression in cells, the influence of environmental factors, or the interaction between the two. In some embodiments, the phenotype is growth, differentiation, and/or maturation. In some embodiments, the phenotype is an inhibition of growth or proliferation. In some embodiments, the phenotype is persistent in vivo. In some embodiments, the phenotype is death. In some embodiments, the phenotype is the effector function of immune cells (e.g., cytokine release and/or cytotoxic killing), or a reduction or absence of effector function.

As used herein, the term "stimulus" refers to a primary response induced by connecting parts of the cell surface. For example, in the context of receptors, such stimulation requires receptor connectivity and subsequent signaling events. Regarding the stimulation of T cells, this stimulation refers to the connection of the surface portion of the T cell, which in one embodiment subsequently induces signal signaling events, such as binding to the TCR/CD3 complex. In addition, stimulating events can activate cells and upregulate or downregulate the expression or secretion of molecules, such as downregulating TGF-β. Therefore, even in the absence of direct signaling events, the connection of cell surface regions can lead to the reorganization of cytoskeleton structures or the aggregation of cell surface regions, each of which can be used to enhance, modify, or alter subsequent cellular responses.

As used herein, the term "activation" refers to the state of a cell after it has fully connected its surface portions to induce significant biochemical or morphological changes. In the context of T cells, this activation refers to a state in which T cells have been adequately stimulated to induce cell proliferation. Activation of T cells can also induce the production and regulation of cytokines or the function of cell lysis effectors. In the context of other cells, the term infers the upregulation or downregulation of a particular physicochemical process. The term "activated T cell" refers to a T cell that is currently undergoing cell division, cytokine production, regulatory function or cell lysis effector function, and/or has recently undergone an "activation" process.

The "isolated" nucleic acid molecules described herein are nucleic acid molecules identified and isolated from at least one contaminating nucleic acid molecule that is typically associated with the environment in which it was produced. Preferably, the isolated nucleic acids do not bind to any components related to the production environment. The isolated nucleic acid molecules encoding the polypeptides herein are in a form different from the form or environment in which they are found in nature. Therefore, the isolated nucleic acid molecules are different from the nucleic acids that encode the polypeptides naturally present in cells.

Unless otherwise stated, a nucleotide sequence "encoding an amino acid sequence" comprises all nucleotide sequences that are degenerate forms of each other and encode the same amino acid sequence. Nucleotide sequences that encode proteins or RNA can also comprise introns, to the extent that the nucleotide sequence encoding a protein may comprise one or more introns in some versions.

As used herein, the terms "transfection" or "transformation" or "transduction" refers to the process of transferring or introducing exogenous nucleic acids into host cells (e.g., immune cells). "Transfected," "transformed," or "transduced" cells are cells that have been transfected, transformed, or transduced with exogenous nucleic acids. The cells comprise primary test cells and their progeny.

As used herein, the term "CAR," or chimeric antigen receptor, comprises: i) an extracellular antigen-binding domain that specifically recognizes one or more target antigens (e.g., tumor antigens) or target epitopes (e.g., tumor epitopes); ii) a transmembrane domain; and iii) an intracellular signaling domain. In some embodiments, the extracellular antigen-binding domain is one or more selected from the group consisting of: the extracellular domain of a ligand, a single-domain antibody (sdAb), a single-chain Fv (scFv), and Fab. In some embodiments, the transmembrane domain is derived from a molecule selected from the group consisting of: TCRα, TCRβ, TCRγ, TCRδ, CD3ζ, CD3ε, CD3γ, CD3δ, CD4, CD5, CD6, CD7, CD8α, CD9, CD16, CD22, CD27, CD28, CD33, CD37, CD45, CD47, CD52, CD64, CD80, CD86, CD134, 4-1BB, CD152, CD154, CISH, and PD-1. In some embodiments, the transmembrane domain is derived from CD8α. In some embodiments, the intracellular signaling domain comprises a primary intracellular signaling domain derived from a molecule selected from the group consisting of: CD3ζ, CD3γ, CD3ε, CD3δ, FcRγ, FcRβ, CD5, CD22, CD79a, CD79b, CD66d, FcγRIIa, DAP10, and DAP12. In some embodiments, the primary intracellular signaling domains is derived from CD3ζ. In some embodiments, the intracellular signaling domain further comprises a co-stimulatory signaling domain derived from one or more co-stimulatory molecules selected from the group consisting of: CARD11, CD2, CD4, CD7, CD19, CD27, CD28, CD30, CD40, CD160, ICAM-1, OX40, 4-1BB, SELPLG, LIGHT, HVEM, B7-H3, ICOS, PD-1, SLAMF7, LFA-1, NKG2C, CDS, GITR, BAFFR, NKp80, IPO-3, SLAMF8, LTBR, LAT, GADS, SLP-76, PAG/Cbp, NKp44, NKp30, NKp46, NKG2D, CD83, SLAMF1, CTLA-4, LAG-3, PD-L2, PD-L1, DAP10, TRIM, ZAP70, ligands that specifically bind to CD83, and any combination thereof. In some embodiments, the co-stimulatory signaling domain is derived from 4-1BB. In some embodiments, the CAR further comprises a hinge domain located between the C-terminus of the extracellular antigen-binding domain and the N-terminus of the transmembrane domain. In some embodiments, the hinge domain is derived from CD8α or CD28.

As used herein, an engineered "TCR" is an engineered T-cell receptor comprising: (a) an extracellular ligand-binding domain comprising an antigen-binding fragment (e.g., sdAb, scFv, Fab, DARPin) that specifically recognizes one or more target antigens (e.g., tumor antigens) or target epitopes (e.g., tumor epitopes); (b) optionally a first adapter; (c) optionally an extracellular domain of a first TCR subunit (e.g., Cα, Cβ, Cδ, Cγ, CD3ε) or a portion thereof; (d) a transmembrane domain of a second TCR subunit (e.g., TCRα, TCRβ); and (e) an intracellular signaling domain comprising an intracellular signaling domain of a third TCR subunit (e.g., TCRα, TCRβ); wherein the first, second, and third TCR subunits are independently selected from any one of the group consisting of: TCRα, TCRβ, TCRγ, TCRδ, CD3ε, CD3γ, CD3δ, and CD3ζ. In some embodiments, the first, second, and third TCR subunits are the same (e.g., all CD3ε, all TCRα, or all TCRβ). In some embodiments, the first, second, and third TCR subunits are different. In some embodiments, the engineered TCR further comprises a hinge domain located between the C-terminus of the extracellular ligand-binding domain and the N-terminus of the transmembrane domain. In some embodiments, the hinge domain is derived from CD8α.

As used herein, "TAC" refers to a T-cell antigen conjugate comprising (i) an antigen-binding domain, (ii) a TCR-binding domain (e.g., scFv), and (iii) a co-receptor domain (e.g., a hinge, transmembrane, and/or cytosol region). See, for example, Helsen et al. Nat Commun. 2018; 9(1):3049. In some embodiments, the TAC comprises: (a) an extracellular ligand-binding domain comprising an antigen-binding fragment (e.g., sdAb, scFv, Fab, DARPin) that specifically recognizes one or more target antigens (e.g., tumor antigens) or target epitopes (e.g., tumor epitopes); (b) optionally a first adapter; (c) an extracellular TCR-binding domain (e.g., sdAb, scFv, Fab, DARPin) that specifically recognizes an extracellular domain of a TCR subunit (e.g., CD3ε); (d) optionally a second adapter; and (e) optionally, an extracellular domain of a first TCR coreceptor (e.g., CD4, CD8) or a portion thereof; (f) a transmembrane domain comprising a transmembrane domain of a second TCR co-receptor (e.g., CD4, CD8); and (g) optionally an intracellular signaling domain comprising an intracellular signaling domain of a third TCR co-receptor (e.g., CD4, CD8); wherein the TCR subunits are one or more selected from the group consisting of: CD3ε, CD3δ, CD3γ, TCRα, TCRβ, TCRγ, and TCRδ; and wherein the first, second, and third TCR co-receptors are independently selected from any one of the group consisting of: CD4, CD8, and CD28. In some embodiments, the first, second, and third TCR co-receptors are the same. In some embodiments, the first, second, and third TCR co-receptors are different. In some embodiments, the TAC further comprises a hinge domain (e.g., derived from CD8α) located between the C-terminus of the extracellular ligand-binding domain and the N-terminus of the transmembrane domain.

As used herein, "treatment" is a method used to obtain beneficial or desired results, including clinical outcomes. For the purposes of this application, beneficial or desired clinical outcomes include, but are not limited to, one or more selected from the group consisting of: relief of one or more symptoms caused by the disease, reduction of the severity of the disease, stabilization of the disease (e.g., prevention or delay of disease exacerbation), prevention or delay of disease spread (e.g., metastasis), prevention or delay of disease recurrence, delay or slowing of disease progression, improvement of disease status, provision of remission (partial or complete), reduction of the dosage of one or more other medications required to treat the disease, delay of disease progression, improvement of quality of life and/or prolongation of survival. "Treatment" also comprises reducing the pathological consequences of cancer or immune diseases.

As used herein, the term "effective amount" refers to an amount of agent (such as modified immune cells or pharmaceutical compositions thereof) sufficient to treat a particular disorder, condition, or disease, such as improving, alleviating, reducing, and/or delaying one or more of its symptoms (e.g., cancer, infectious diseases, GvHD, transplant rejection, autoimmune diseases, or radiation sickness). When cancer is mentioned, an effective amount comprises an amount sufficient to shrink the tumor and/or slow its growth rate (such as inhibiting tumor growth) or to prevent or delay the proliferation of other unwanted cells. In some embodiments, the effective amount is the amount sufficient to delay development. In some embodiments, the effective amount is an amount sufficient to prevent or delay recurrence. The effective amount can be administered in one or more administrations. An effective amount of an agent (e.g., modified immune cells) or a composition may: (i) reduce the number of cancer cells; (ii) reduce tumor size; (iii) inhibit, delay, slow down, and preferably prevent cancer cell infiltration into peripheral organs to a certain extent; (iv) inhibit (i.e., slow down and preferably stop) tumor metastasis to a certain extent; (v) inhibit tumor growth; (vi) prevent or delay the occurrence and/or recurrence of tumors; and/or (vii) alleviate one or more symptoms associated with cancer to a certain extent. In the case of infectious diseases, such as viral infections, therapeutically effective amounts of the modified immune cells or compositions thereof described herein can reduce the number of cells infected by pathogens; reduce the production or release of pathogen-derived antigens; inhibit (i.e., to some extent slow down and preferably stop) the spread of pathogens to uninfected cells; and/or to some extent alleviate one or more symptoms associated with the infection. In some implementation schemes, the effective therapeutic amount is the amount that prolongs the patient's survival.

As used herein, "individual" or "subject" refers to mammals, including but not limited to: humans, cattle, horses, felines, canines, rodents, or primates. In some embodiments, the individual is a person.

As used herein, "patient" comprises anyone who has a disease (such as cancer). The terms "subject," "individual," and "patient" are used interchangeably herein.

The term "multiple of infection" or "MOI" is used interchangeably herein and refers to the ratio of a pathogen (e.g., a bacteriophage, virus, or bacterium) to its target of infection (e.g., a cell or organism). For example, when referring to a group of cells inoculated with viral particles, the multiplicity of infection (MOI) refers to the ratio between the number of viral particles (e.g., viral particles comprising a library of sgRNAs) during viral transduction and the number of target cells present in the mixture.

When the term "comprising" is used in this specification and claims, other elements or steps are not excluded.

It should be understood that the embodiments of this application described herein comprise embodiments that are "composed of" and/or "substantially composed of".

When this article refers to a value or parameter "about", it comprises (and describes) the changes that occur with respect to that value or parameter itself. For example, a description involving "about X" comprises a description of "X".

As used herein, mentioning "not" a value or parameter usually means and describes "except" for that value or parameter. For example, the statement that the method is not used to treat type X cancer means that the method is used to treat other types of cancer besides type X.

The term "about X-Y" used herein has the same meaning as "from about X to about Y".

In the description of the numerical range of nucleotides herein, each intermediate number is explicitly considered. For example, for the range of 19-21nt, the number 20nt is considered in addition to 19nt and 21nt, and for the range of MOI, every intermediate number in between is explicitly considered, whether it is an integer or a decimal.

As used herein and in the appended claims, unless the context clearly specifies otherwise, the singular forms "a," "an," and "the" refer to the plural objects.

Those skilled in the art will understand that uracil and thymine can both be represented by "t" instead of "u" for uracil and "t" for thymine; in the context of ribonucleic acid, unless otherwise stated, it will be understood that "t" represents uracil.

Unless otherwise specified, in this application, when referring to genomic loci of human genes, the reference genome used is GRCh38.p14.

Engineered T cells

On the one hand, this application provides an engineered T cell that is modified to eliminate or reduce (e.g., reduce by any one of at least about 10%, 20%, 30%, 40%, 50%, 60%, 70%, 80%, 90%, 95% or more) the expression and/or function of endogenous T cell receptor (TCR) protein and/or SPPL3 protein. In some embodiments, the T cells are $\alpha\beta$T cells that are modified to eliminate or reduce the expression and/or function of the $\alpha$ subunit (TCR$\alpha$) and/or $\beta$ subunit (TCR$\beta$) of the endogenous T cell receptor (TCR). In some embodiments, the T cells are $\alpha\beta$T cells, and the expression and/or function of endogenous TCR$\alpha$ and/or TCR$\beta$ or a functional fragment thereof in the $\alpha\beta$T cells is eliminated or reduced. In some embodiments, the T cells are $\gamma\delta$T cells that are modified to eliminate or reduce the expression and/or function of the endogenous $\gamma$ subunit (TCR$\gamma$) and/or T cell receptor (TCR) $\delta$ subunit (TCR$\delta$).

In some embodiments, the engineered T cells have normally expressed HMC class I proteins. In some embodiments, the engineered T cells have normal expression of B2M, HLA-A, HLA-B, and HLA-C. In some embodiments, the engineered T cells are human T cells and have normally expressed HLA class I proteins.

In some embodiments, compared with T cells that do not have reduced or eliminated endogenous TCR and/or SPPL3 proteins, the activation-induced cell death (AICD) of engineered T cells is reduced by at least about 10% (e.g., any one of at least about 20%, 30%, 40%, 50%, 60%, 70%, 80%, 90%, 95%, or 100%), for example, by at least about 20%. In some embodiments, compared to engineered T cells that do not have reduced or eliminated expression (RNA and/or protein expression) and/or function of the SPPL3 protein, the AICD of engineered T cells is reduced by at least about 10% (e.g., any one of at least about 20%, 30%, 40%, 50%, 60%, 70%, 80%, 90%, 95%, or 100%), for example, by at least about 20%.

In some embodiments, compared with T cells that do not have reduced or eliminated endogenous TCR and/or SPPL3 protein, the engineered T cells, after transplantation into an allogeneic recipient, exhibit a reduction in HVG caused by allogeneic T cell attack by at least about 10% (e.g., any one of at least about 20%, 30%, 40%, 50%, 60%, 70%, 80%, 90%, 95%, or 100%), for example, by at least about 20%. In some embodiments, compared to engineered T cells that do not have reduced or eliminated expression (RNA and/or protein expression) and/or function of the SPPL3 protein, the engineered T cells exhibit a reduction in HVG caused by allogeneic T cell attack by at least about 10% (e.g., any one of at least about 20%, 30%, 40%, 50%, 60%, 70%, 80%, 90%, 95%, or 100%)for example, by at least about 20%.

In some embodiments, compared to engineered T cells that do not have reduced or eliminated expression (RNA and/or protein expression) and/or function of the endogenous TCR protein and/or SPPL3 protein, the engineered T cells exhibit at least 10% longer in vivo persistence (e.g., any one of at least about 20%, 30%, 40%, 50%, 60%, 70%, 80%, 90%, 1-fold, 1.5-fold, 2-fold, 5-fold, 10-fold, 20-fold, 50-fold or more).

In some embodiments, compared to T cells that do not have reduced or eliminated expression and/or function of MHC class I molecules and TCR proteins, and reduced or eliminated expression and/or function of SPPL3 proteins, the engineered T cells of this application show no significant difference in the degree of HVG caused by allogeneic T cell attack; and compared to T cells that do not have reduced or eliminated expression and/or function of MHC class I molecules and TCR proteins, and reduced or eliminated expression and/or function of SPPL3 proteins, the engineered T cells of this application show at least a 10% reduction in natural killing caused by NK cell attack (e.g., any one of at least about 20%, 30%, 40%, 50%, 60%, 70%, 80%, 90%, 95%, or 100%), for example, a reduction of at least about 20%. Therefore, compared to T cells that do not have reduced or eliminated expression and/or function of MHC class I molecules and TCR proteins, and reduced or eliminated expression and/or function of SPPL3 proteins, in some embodiments, in an in vivo environment, in a peripheral blood environment, in the presence of PBMCs, or in the presence of both NK cells and T cells, the engineered T cells of this application are substantially no difference in terms of the killing effect of NK cells and T cells, or the killing effect is reduced by at least about 10% (e.g., any one of at least about 20%, 30%, 40%, 50%, 60%, 70%, 80%, 90%, 95%, or 100%), for example, reduced by at least about 20%. In some embodiments, compared to T cells that do not have reduced or eliminated expression and/or function of MHC class I molecules and TCR proteins, and reduced or eliminated expression and/or function of SPPL3 proteins, the engineered T cells of this application maintain a substantially consistent duration in allogeneic recipients or allogeneic blood or PBMCs, or have a longer persistence, i.e., they can exist for a time with no significant difference or a longer duration, such as maintaining a half-life with no significant difference or a longer duration in vivo.

In some embodiments, the engineered T cells have or express engineered receptors. In some embodiments, the engineered T cells are further modified to express engineered receptors. In some embodiments, the engineered receptor is one or more selected from the group consisting of: CAR, TCR, and TAC. In some embodiments, the engineered T cells are CAR-T cells, TCR-T cells, or TAC-T cells. In some embodiments, the engineered receptor is monovalent. In some embodiments, the engineered receptor is multivalent. In some embodiments, the engineered receptor is monospecific, for example, monovalent and monospecific, or multivalent and monospecific. In some embodiments, the engineered receptor is multispecific (e.g., bispecific).

In some embodiments, the engineered T cells are modified to reduce or eliminate the expression (RNA and/or protein expression) and/or function of endogenous TCR protein and/or SPPL3 protein and/or other proteins, and/or the immune cells modified to express engineered receptors can be autologous or allogeneic.

Methods for preparing engineered T cells

Secondly, this application also provides a method for preparing the above-mentioned engineered T cells.

In some embodiments, the expression of the endogenous TCR protein and/or SPPL3 protein in the engineered T cells is reduced (e.g., reduced by any one of at least about 10%, 20%, 30%, 40%, 50%, 60%, 70%, 80%, 90%, 95% or more) or inhibited by antisense RNA, miRNA, siRNA or shRNA that specifically recognizes RNA encoding the endogenous TCR and/or SPPL3 protein.

In some embodiments, the function of endogenous TCR and/or SPPL3 proteins is reduced or inhibited by chemically modified mRNAs, such as dominant-negative inhibitors of chemically modified endogenous TCR and/or SPPL3 proteins (e.g., dominant-negative variants or fragments thereof, or dominant-negative binding chaperones). In some embodiments, immune cells are modified to express dominant-negative SPPL3 protein variants or dominant-negative fragments thereof. In some embodiments, immune cells are modified to express the dominant-negative binding chaperone of the SPPL3 protein.

In some embodiments, the expression (RNA and/or protein expression) and/or function of endogenous TCR protein and/or SPPL3 protein is reduced (e.g., reduced by any one of at least about 10%, 20%, 30%, 40%, 50%, 60%, 70%, 80%, 90%, 95% or more) or inhibited by small molecule compounds, nucleic acids (or vectors comprising them), lipids and/or protein molecules.

In some embodiments, the engineered T cells are genetically modified at DNA loci encoding endogenous TCR and/or SPPL3 proteins. In some embodiments, immune cells are genetically modified at the SPPL3 locus. In some embodiments, the loci of the endogenous TCR protein and/or SPPL3 protein are modified with a mutagen. Mutagens can be classified into three categories: physical (e.g., gamma rays, ultraviolet (UV) radiation), chemical (e.g., ethyl methanesulfonate (EMS)), and transposon elements (e.g., transposons, retrotransposons, T-DNA, retroviruses). In some embodiments, the mutagen or condition is ionizing radiation (IR), ultraviolet radiation, alkylating agents (such as nitrogen mustard, methyl methanesulfonate (MMS), EMS, N-ethyl-N-nitrosourea (ENU)), aromatic amines (e.g., 2-aminofluorene), polycyclic aromatic hydrocarbons (PAHs; e.g., dibenzo[a,l]pyrene, naphthalene, anthracene, pyrene), crosslinking, insertional mutagenesis (e.g., mediated by transposons or viruses), or other toxins (e.g., aflatoxin, N-nitrosamines). In some embodiments, the endogenous TCR protein and/or SPPL3 protein loci are modified by gene editing. In some embodiments, gene editing is mediated by site-directed mutagenesis (SDM). In some embodiments, gene editing is mediated by random and extensive mutagenesis (REM). In some embodiments, gene editing is performed using PCR methods. In some embodiments, gene editing is performed using non-PCR methods. Any known gene editing method may be used herein, including but not limited to non-homologous end joining (NHEJ) mediated, homologous directed repair (HDR) mediated, zinc finger nuclease (ZFN) mediated, transcription activator-like effector nuclease (TALEN) mediated, or CRISPR/Cas mediated gene editing. HDR can occur either non-conservatively or conservatively. In some embodiments, HDR is mediated via a single-chain annealing (SSA) pathway. In some embodiments, HDR is mediated via the classical double-strand fracture repair (DSBR) pathway, the synthesis-dependent chain annealing (SDSA) pathway, or the fracture-induced repair (BIR) pathway. In some embodiments, the cell modification methods described herein further comprise introducing a nucleic acid template (e.g., comprising the desired mutation), such as inserting at a double-strand break (DSB), to modify the target genome sequence (e.g., via HDR). Gene editing can introduce one or more mutations into a DNA locus encoding one or more target proteins (e.g., SPPL3 protein), including but not limited to insertion, deletion, substitution (such as nonsynonymous substitution), truncation, translocation, point mutation, etc. In some embodiments, the mutation is a frameshift mutation, a loss-of-function (LOF) mutation, a dominant-negative mutation, a missense mutation, or a nonsense mutation. In some embodiments, gene editing comprises gene knockout (KO). In some embodiments, gene editing comprises base editing (e.g., introducing non-synonymous substitutions). In some embodiments, base editing introduces a stop codon, which can reduce the expression of functional RNA and/or proteins. In some embodiments, base editing introduces mutations that affect RNA and/or protein function. In some embodiments, gene editing is mediated by CRISPR/Cas. In some embodiments, the Cas protein has endonuclease activity. In some embodiments, the Cas protein is a fusion protein comprising i) a dead Cas protein (dCas) and ii) an adenine base editor (ABE) or adenine deaminase (ADA), or a cytidine base editor (CBE) or cytidine deaminase (CDA), or a functional fragment thereof. Cytidine base editors can convert target C:G base pairs to T:A base pairs, while adenine base editors can convert A:T base pairs to G:C base pairs. In short, these two types of base editors can target and install all possible conversion mutations (C to T, G to A, A to G, T to C, C to U, and A to U).

In some embodiments, the engineered T cells are genetically modified at the RNA encoding endogenous TCR and/or SPPL3 proteins. In some embodiments, the RNA encoding the endogenous TCR protein and/or SPPL3 protein is modified by RNA editing. RNA editing can introduce one or more mutations into RNA encoding endogenous TCR and/or SPPL3 proteins, including but not limited to insertion, deletion, substitution (such as nonsynonymous substitution), truncation, point mutation, etc. In some embodiments, the mutation is a frameshift mutation, a LOF mutation, a dominant-negative mutation, a missense mutation, or a nonsense mutation. In some embodiments, RNA editing comprises base editing (e.g., introducing non-synonymous substitutions, such as C to U, A to I). Any known RNA editing method may be used herein (see, for example, Guillermo Aquino-Jarquin, "Novel Engineered Programmable Systems for ADAR-Mediated RNA Editing," Mol Ther Nucleic Acids. 2020; 19:1065-1072; the contents of which are incorporated herein by reference in their entirety), including but not limited to programmed RNA editing using endogenous ADAR ("LEAPER"; see, for example, WO2020074001 and Qu et al.). Nat Biotechnol. 2019; 37(9):1059-1069), or programmed A-to-I substitution RNA editing ("REPAIR"; see, for example, Cox et al., "RNA editing with CRISPR-Cas13," Science. 2017; 358(6366):1019-1027), oligonucleotide-mediated RNA editing by recruiting endogenous ADARs to specific transcripts ("RESTORE"; see, for example, Merkle et al., "Precise RNA editing by recruiting endogenous ADARs with antisense oligonucleotides," Methods Mol Biol. 2021; 2181:331-349), CRISPR-Cas-triggered RNA targeting systems ("CIRTS"; see, for example, Rauch et al., "Programmable RNA-Guided RNA Effector Proteins Built from Human Parts,"Cell. 2019; 178(1):122-134.e12", RNA editing for specific C-U exchange ("RESCUE"; see, for example, Abudayyeh et al., "A cytosine deaminase for programmable single-base RNA editing," Science. 2019; 365(6451):382-386), or CLUSTER (see, for example, P. Reautschnig et al., "CLUSTER guide RNAs enable precise and efficient RNA editing with endogenous ADAR enzymes in vivo," Nat Biotechnol. 2022 May; 40(5):759-768), the contents of each of which are incorporated herein by reference in their entirety. In some embodiments, RNA editing is mediated by LEAPER. In some embodiments, RNA editing is mediated by CRISPR/Cas, such as by fusing an adenine base editor (ABE) or adenine deaminase (ADA), or a cytidine base editor (CBE) or cytidine deaminase (CDA), or a functional fragment thereof, with a dead Cas (dCas, e.g., dCas13) protein.

Methods for creating CRISPRs that identify predetermined DNA or RNA sites are known in the art. Any known CRISPR/Cas system suitable for gene editing or RNA editing can be used herein. To date, based on the outstanding functionality and evolutionary modularity of CRISPR-Cas systems, two classes (class 1 and class 2) and six types (I-VI) of CRISPR-Cas systems have been described. See, for example, Nidhi et al., "Novel CRISPR-Cas Systems: An Updated Review of the Current Achievements, Applications, and Future Research Perspectives," Int J Mol Sci. 2021; 22(7):3327, the contents of which are incorporated herein by reference in their entirety. Among the two types of CRISPR-Cas systems, the type II Cas9 system and the V-A/B/E/J type Cas12a/Cas12b/Cas12e/Cas12j system have been used for genome editing, providing broad prospects for biomedical research. Cas13a (C2c2) is a VI-A type RNA-guided RNA-targeting CRISPR effector that can be used for RNA editing as described herein. The CRISPR/Cas system used herein can generate double-strand breaks (DSBs) or single-strand breaks at predetermined nucleic acid sites. In some embodiments, the CRISPR/Cas system used herein is the CRISPR/Cas9 system. In some embodiments, the CRISPR/Cas system used herein is the CRISPR/Cas12 system.

In some embodiments, gene editing or RNA editing comprises, under conditions allowing the introduction of a gRNA construct and optionally a Cas component into precursor T cells, contacting the precursor T cells with: i) a guide RNA (gRNA) construct comprising or encoding a gRNA comprising a guide sequence complementary to a target in a DNA locus or RNA encoding an endogenous TCR protein and/or SPPL3 protein; and optionally ii) a Cas component comprising a Cas protein or nucleic acid encoding a Cas protein. In some embodiments, the precursor immune cells have expressed the Cas protein before the gRNA construct is introduced. In some embodiments, the precursor immune cells do not express the Cas protein before the introduction of the gRNA construct and Cas components. In some embodiments, the progenitor immune cells express engineered receptors (e.g., CAR, engineered TCR, or TAC) prior to the introduction of the gRNA construct and/or Cas component. In some embodiments, the Cas protein has endonuclease activity. In some embodiments, the Cas protein is a fusion protein, such as a fusion protein comprising i) dCas (e.g., dCas13a, dCas9) and ii) ADA (e.g., TadA, such as TadA8e) or CDA or a functional fragment thereof. In some embodiments, the Cas protein is Cas9, such as dCas9. In some embodiments, the gRNA is sgRNA. In some embodiments, the gRNA comprises (or substantially consists of, or consists of) crRNA. In some embodiments, the gRNA comprises (or substantially consists of, or consists of) crRNA and tracrRNA.

In some embodiments, immune cells modified to eliminate or reduce (e.g., reduce any one of at least about 10%, 20%, 30%, 40%, 50%, 60%, 70%, 80%, 90%, 95% or more) endogenous TCR protein and SPPL3 have been modified or further modified to eliminate or reduce (e.g., reduce any one of at least about 10%, 20%, 30%, 40%, 50%, 60%, 70%, 80%, 90%, 95% or more) the expression (RNA and/or protein expression) and/or function of one or more other proteins selected from the group consisting of: PD-1, TIM-3, LAG-3, CTLA-4, CISH, SPPL3, Fas, FADD, CASP8, ARID1A, BAK1, BID, ETS1, IKZF2, HIST1H1B, B7-H6, MICA, MICB, ULBP1, ULBP2, ULBP3, ULBP4, ULBP5, ULBP6, and ligands of NKp46. In some embodiments, the T cells have normally expressed HLA-I, which does not lead to NK cell killing.

In some embodiments, the engineered T cells are further modified to express engineered receptors. In some embodiments, immune cells have expressed engineered receptors before being modified to reduce or eliminate the expression and/or function of endogenous TCR and/or SPPL3 proteins. In some embodiments, immune cells are modified to reduce or eliminate the expression and/or function of endogenous TCR protein and/or SPPL3 protein, and are further modified to express engineered receptors. In some embodiments, modifications that reduce or eliminate the expression and/or function of endogenous TCR proteins and/or SPPL3 proteins, as well as modifications that express engineered receptors, occur simultaneously. In some embodiments, the reduction or elimination of the expression and/or function of endogenous TCR protein and/or SPPL3 protein, and the modification to express engineered receptors occur sequentially. Any engineered receptor capable of transducing signals to immune cells (e.g., inducing cell proliferation, cytokine production, and/or performing regulatory or cytolytic effector functions) and/or recognizing target antigens may be used herein. In some embodiments, the engineered receptor is a chimeric antigen receptor (CAR), an engineered TCR, or a T-cell antigen conjugate (TAC). In some embodiments, the engineered receptor is monovalent. In some embodiments, the engineered receptor is multivalent. In some embodiments, the engineered receptor is monospecific, for example, monovalent and monospecific, or multivalent and monospecific. In some embodiments, the engineered receptor is multispecific (e.g., bispecific).

In some embodiments, modifications that reduce or eliminate the expression (RNA and/or protein expression) and/or function of endogenous TCR and/or SPPL3 proteins do not downregulate or eliminate the expression (RNA and/or protein expression) and/or function of engineered receptors (e.g., CARs). In some embodiments, modifications that reduce or eliminate the expression and/or function of endogenous TCR and/or SPPL3 proteins downregulate the expression (RNA and/or protein expression) and/or function of engineered receptors by up to about 30% (e.g., any one of up to about 25%, 20%, 15%, 10%, 5%, 1% or less).

In some embodiments, engineered receptors (e.g., CAR), gRNA constructs or arRNA constructs and/or Cas components are introduced into precursor T cells by transducing/transfecting nucleic acids (DNA or RNA) or vectors encoding them (e.g., non-viral vectors, or viral vectors such as lentiviral vectors), or viruses comprising nucleic acids encoding them (e.g., lentiviruses). In some embodiments, the Cas component (e.g., Cas9 protein) is introduced into precursor T cells by inserting the protein into the cell membrane, while the cells are introduced via a microfluidic system, such as CELL SQUEEZE® (see, for example, U.S. Patent Application No. 20140287509).

Methods for introducing vectors (e.g., viral vectors) or isolated nucleic acids into mammalian cells are known in the art. The nucleic acids or vectors described herein can be transferred to T cells using physical, chemical, or biological methods. Physical methods for introducing vectors (e.g., viral vectors) into cells comprise calcium phosphate precipitation, lipid transfection, gene gun method, microinjection, electroporation, etc. Methods for producing cells comprising vectors and/or exogenous nucleic acids are well known in the art. In some embodiments, a vector (e.g., a viral vector) is introduced into the cell via electroporation. Biological methods for introducing vectors into cells comprise the use of DNA and RNA vectors. Chemical means of introducing a vector (e.g., a viral vector) into cells comprise colloidal dispersion systems, such as macromolecular complexes, nanocapsules, microspheres, beads, and lipid-based systems, including oil-in-water emulsions, micelles, mixed micelles, and liposomes. An exemplary colloidal system used as an in vitro delivery vector is a liposome (e.g., an artificial membrane vesicle).

In some embodiments, RNA molecules (e.g., gRNA, arRNA, or mRNA encoding Cas) are prepared by conventional methods (e.g., in vitro transcription) and then introduced into T cells by known methods such as electroporation. In some embodiments, a viral vector (lentiviral vector) or virus (e.g., lentivirus) comprising nucleic acid encoding any engineered receptor (e.g., CAR), gRNA or arRNA and/or the Cas protein described herein is contacted with the precursor T cell at an MOI of at least about 1, such as any one of at least about 1, 1.5, 2, 2.5, 3, 3.5, 4, 4.5, 5, 5.5, 6, 6.5, 7, 8, 9 or 10 (e.g., an MOI of about 3).

In some embodiments, transduced/transfected T cells are proliferated in vitro after the introduction of a vector or isolated nucleic acid. In some embodiments, the transduced/transfected T cells are cultured to proliferate for any one of at least about 1 day, 2 days, 3 days, 4 days, 5 days, 6 days, 7 days, 10 days, 12 days, or 14 days, such as 7 days. In some embodiments, the transduced/transfected T cells are further evaluated or screened to purify the T cells described in this application.

Reporter genes can be used to identify potentially transfected/transduced cells and to assess the function of regulatory sequences. Generally speaking, a reporter gene is a gene that is not present in or expressed by a recipient organism or tissue and encodes a polypeptide, and the expression of the polypeptide is demonstrated by some easily detectable property, such as enzyme activity. After introducing DNA/RNA into recipient cells, the expression of reporter genes is measured at an appropriate time. Suitable reporter genes may comprise genes encoding luciferase, β-galactosidase, chloramphenicol acetyltransferase, secretory alkaline phosphatase, or green fluorescent protein (GFP) (e.g., Ui-Tei et al. FEBS Letters 479:79-82 (2000)). Suitable expression systems are well known and can be prepared or commercially obtained using known techniques. Antibiotic selection markers can also be used to identify potentially transfected/transduced cells.

Other methods for confirming the presence of any nucleic acid (e.g., gRNA construct or sgRNA construct) or mutation (e.g., inactivation mutation) described herein in the target genes of the engineered T cells of this application comprise, for example, molecular biological assays well known to those skilled in the art, such as Southern and Northern blotting, RT-PCR, PCR, DNA-seq, or RNA-seq; and biochemical analyses, such as detecting the presence or absence of a specific peptide by immunological methods (e.g., ELISA and Western blotting), fluorescence-activated cell sorting (FACS), or magnetically activated cell sorting (MACS).

**Methods for prolonging the in vivo half-life of engineered cells**

Typically, after allogeneic engineered cells enter the patient's body, they are often cleared from the donor due to factors such as GvHD, AICD, and HVG, resulting in poor persistence in vivo and insufficient time to exert their effects in the patient's body, thus limiting clinical efficacy. Common practices comprise knocking out HMC class I molecules in allogeneic engineered cells to prevent HVG. However, knocking out MHC class I molecules can lead to NK cells recognizing and attacking cells that downregulate HHC class I molecules.

This application provides a novel method for prolonging the in vivo half-life of engineered T cells. This method can eliminate HVG while avoiding or reducing NK cell attack caused by the reduction in the amount of MHC class I molecules, thereby prolonging the in vivo half-life of engineered cells by at least about 10% (e.g., any one of at least about 20%, 30%, 40%, 50%, 60%, 70%, 80%, 90%, 1, 1.5, 2, 5, 10, 20, 50 or more) relative to un-engineered T cells. Furthermore, in some embodiments, the method of this application can achieve similar or better results than the method for prolonging the in vivo half-life of engineered cells by eliminating or reducing MHC class I molecules. In some embodiments, compared to the method for prolonging the half-life of engineered cells by eliminating or reducing MHC class I molecules, the method of the present application can achieve the same degree of HVG received by the engineered T cells, but the natural killing caused by NK cell attacks received is reduced by at least about 10% (e.g., at least about 20%, 30%, 40%, 50%, 60%, 70%, 80%, 90%, 95%, or 100%), for example, by at least about 20%.

In some embodiments, the engineered cells are immune cells or precursor cells thereof. In some embodiments, the immune cells are one or more selected from the group consisting of: T cells, B cells, NK cells, macrophages, and DC cells. In some embodiments, the immune cells are T cells. In some embodiments, the immune cells are T cells, and they are modified to eliminate or reduce the expression and/or function of endogenous TCR proteins. In some embodiments, the engineered cells express or have one or more engineered receptors selected from the group consisting of: CAR, TCR, and TAC. In some embodiments, the engineered T cells are CAR-T cells, TCR-T cells, or TAC-T cells. In some embodiments, the engineered receptor is monovalent. In some embodiments, the engineered receptor is multivalent. In some embodiments, the engineered receptor is monospecific, for example, monovalent and monospecific, or multivalent and monospecific. In some embodiments, the engineered receptor is multispecific (e.g., bispecific).

Specifically, the method comprises modifying the engineered cells to eliminate or reduce the expression and/or function of SPPL3 protein or a functional fragment thereof. In some embodiments, the method comprises any of the methods for preparing engineered T cells described above.

Guide RNA

This application also provides a guide RNA (e.g., sgRNA) for the CRISPR/Cas system and constructs encoding the CRISPR/Cas guide RNA to hit the SPPL3 gene by cutting or base editing and to generate mutations in SPPL3, especially frameshift mutations, referred to as gRNA targeting SPPL3. Using the sgRNA provided in this application to knock out SPPL3 in engineered cells, and using SPPL3 as a marker to screen engineered cells that do not express SPPL3, the SPPL3 knockout efficiency of the screened engineered cells still reach 99.37% after several days (e.g., 1, 2, 3, 4, 5, 6, 7, or 8 days) of culture. Furthermore, the guide RNA provided in this application does not cause detectable off-target effects. In some embodiments, the guide RNA provided in this application comprises a guide sequence as shown in any one of SEQ ID NO:6 to SEQ ID NO:256 or SEQ ID NO:261 to SEQ ID NO:316. In some embodiments, the guide RNA provided in this application comprises a guide sequence as shown in any one of SEQ ID NO:68, SEQ ID NO:145, SEQ ID NO:150, SEQ ID NO:154, SEQ ID NO:155, SEQ ID NO:159, SEQ ID NO:161, SEQ ID NO:165, SEQ ID NO:172, SEQ ID NO:176, SEQ ID NO:196, SEQ ID NO:203, SEQ ID NO:204, SEQ ID NO:209, SEQ ID NO:212, SEQ ID NO:241, SEQ ID NO:250, SEQ ID NO:261, SEQ ID NO:269, SEQ ID NO:270, SEQ ID NO:271, SEQ ID NO:272, SEQ ID NO:273, SEQ ID NO:274, SEQ ID NO:275, SEQ ID NO:276, SEQ ID NO:277, SEQ ID NO:278, SEQ ID NO:279, SEQ ID NO:280, SEQ ID NO:281, SEQ ID NO:282, SEQ ID NO:283, SEQ ID NO:284, SEQ ID NO:285, SEQ ID NO:286, SEQ ID NO:287, SEQ ID NO:288, SEQ ID NO:289, SEQ ID NO:290, SEQ ID NO:291, SEQ ID NO:292, SEQ ID NO:293, SEQ ID NO:294, SEQ ID NO:295, SEQ ID NO:297, SEQ ID NO:300, SEQ ID NO:305, SEQ ID NO:312, and SEQ ID NO:315. In some embodiments, the guide RNA provided in this application comprises a guide sequence as shown in any one of SEQ ID NO:150, SEQ ID NO:154, SEQ ID NO:155, SEQ ID NO:159, SEQ ID NO:161, SEQ ID NO:269, SEQ ID NO:270, SEQ ID NO:271, SEQ ID NO:272, SEQ ID NO:273, SEQ ID NO:274, SEQ ID NO:275, SEQ ID NO:276, SEQ ID NO:277, SEQ ID NO:278, SEQ ID NO:279, SEQ ID NO:280, SEQ ID NO:281, SEQ ID NO:282, SEQ ID NO:283, SEQ ID NO:284, SEQ ID NO:285, SEQ ID NO:286, SEQ ID NO:287, SEQ ID NO:288, SEQ ID NO:289, SEQ ID NO:290, SEQ ID NO:291, SEQ ID NO:292, SEQ ID NO:293, and SEQ ID NO:294. In some embodiments, this application also provides a combination of guide RNAs comprising the above-mentioned gRNA targeting SPPL3 and gRNA targeting the TCR gene. The TCR gene is one or more selected from the group consisting of: TCRα, TCRβ, TCRγ, and TCRδ. In some embodiments, the gRNA targeting the TCR gene targets the TRAC gene.

In this application, gRNA targeting a certain gene refers to the complementary binding of the guide sequence in the gRNA to the coding and/or non coding regions of the gene, or to the mRNA or pre mRNA of the gene.

Depending on the CRISPR/Cas system, gRNA may comprise other components besides the guide sequence. The other components comprise, for example, additional sequence elements that promote the formation of a CRISPR complex with the Cas protein. In some embodiments, the gRNA (e.g., sgRNA) comprises a second sequence comprising a repeat-anti-repeat stem-loop. The repeat-anti-repeat stem loop comprises a tracr-paired sequence fused with a tracr sequence, the tracr sequence being complementary to the tracr-paired sequence through the loop region. In some embodiments, gRNA (e.g., sgRNA) comprises crRNA comprising a direct repeat (DR) sequence capable of interacting with the Cas protein. In some embodiments, the gRNA (e.g., sgRNA) comprises crRNA and tracrRNA, which may be on the same RNA strand (e.g., forming sgRNA) or on two RNA strands. The DR sequence can be derived from the DR sequence naturally associated with the corresponding Cas protein. In some embodiments, the DR sequence is located at the 5' end of the spacer sequence. In some embodiments, the DR sequence is located at the 3' end of the spacer sequence. In some embodiments, the DR sequence comprises one or more mutations relative to a reference (e.g., wild-type) DR sequence, such as one or more mutations including 5' and/or 3' extensions, 5' and/or 3' truncations, nucleotide insertions, nucleotide deletions, nucleotide substitutions, or combinations thereof compared to the reference DR sequence. In some embodiments, the DR sequence comprises both repeat and anti-repeat sequences. In some embodiments, the repeat sequence of the DR sequence is partially or completely complementary to and hybridizes with the anti-repeat sequence of the DR sequence to form a repeat: anti-repeat double strand (also known as a "stem"). In some embodiments, the length of the repeating anti-repeat double strand is about 6 to 20 nucleotides, such as any one of about 6, 8, 10, 12, 14, 16, 18, or 20 nucleotides, or longer. In some embodiments, the repeat sequence of the DR sequence and the anti-repeat sequence of the DR sequence are directly or indirectly connected by an adapter, such as a loop sequence. When repeat and anti-repeat sequences are indirectly connected by an adapter, the length of the repeat: an adapter double strand does not comprise the length of the adapter. In some embodiments, the repeat sequence of the DR sequence and the anti-repeat sequence of the DR sequence are indirectly connected by a loop sequence. In some embodiments, the length of the loop sequence is from about 4 to about 10 nucleotides, for example, any one of about 4, 5, 6, 7, 8, 9 or 10 nucleotides. In some embodiments, the loop sequence comprises the sequence of GAA. In some embodiments, the DR sequence of the gRNA comprises a stem loop. In some embodiments, the DR sequence comprises one or more stem loops, such as about 1 to about 5 stem loops.

Typically, in the context of an endogenous CRISPR/Cas9 system, the formation of a CRISPR complex (comprising a guide sequence that hybridizes with the target sequence and complexes with one or more Cas proteins) results in the cleavage of one or both strands within or near the target sequence (e.g., within 1, 2, 3, 4, 5, 6, 7, 8, 9, 10, 20, 50 or more base pairs). The tracr sequence, which may comprise all or part of or consist of the wild-type tracr sequence (e.g., any one of about 20, 26, 32, 45, 48, 54, 63, 67, 85 or more nucleotides of the wild-type tracr sequence), may also form part of the CRISPR complex, such as by hybridization along at least part of the tracr sequence with all or part of the tracr pairing sequence operatively linked to the guide sequence. In some embodiments, the tracr sequence is sufficiently complementary to the tracr pairing sequence to hybridize and participate in the formation of the CRISPR complex. Similar to the target sequence, it is believed that complete complementarity is not required, as long as sufficient functionality is achieved. In some embodiments, when optimally aligned, the tracr sequence has any one of at least about 50%, 60%, 70%, 80%, 90%, 95%, or 99% sequence complementarity along the length of the tracr paired sequence. Determining the optimal arrangement is within the capabilities of those skilled in the art. For example, there are publicly available and commercially available alignment algorithms and programs, such as, but not limited to: ClustalW, Smith-Waterman in Matlab, Bowtie, Geneious, Biopython, and SeqMan. In some embodiments, the length of the tracr sequence is any one of about or more than about 5, 6, 7, 8, 9, 10, 11, 12, 13, 14, 15, 16, 17, 18, 19, 20, 25, 30, 40, 50 or more nucleotides. Any known tracr pairing sequence and tracr sequence from naturally occurring CRISPR systems can be used, such as the tracr pairing sequence and tracr sequence from the *Streptococcus pyogenes* CRISPR/Cas9 system, as those described in US8697359 and herein.

In some embodiments, the tracr sequence and the tracr pairing sequence are comprised within a single transcript, such that hybridization between the two produces a transcript with a secondary structure, such as a stem-loop (also known as a hairpin), referred to as a "repeat-anti-repeat stem-loop".

"Stem-loop" refers to a nucleic acid with a secondary structure that comprises nucleotide regions that are known or predicted to pair and form a double helix (stem portion) linked by unpaired single-stranded nucleotide regions (loop portion). The terms "hairpin," "hairpin loop," and "return" structure are also used herein to refer to the stem loop. Such structures are well known in the are, and these terms are used according to their well-known meanings in the art. As known in the art, stem-loop structures do not require precise base pairing. Therefore, a stem can comprise one or more base mismatches. In addition, base pairing can be precise, meaning there are no mismatches.

In some embodiments, the sgRNA comprises at least one, two or more stem loops, such as 2, 3, 4 or 5 stem loops. In some embodiments, the sgRNA has up to 5 hairpins. In some embodiments, the sgRNA construct also comprises a transcription termination sequence, such as a polyT sequence, for example, 6 T nucleotides.

In some embodiments, the Cas protein is Cas9 or Cas12, and each sgRNA comprises a guide sequence fused to a second sequence, the second sequence comprising a repeat-anti-repeat stem-loop that interacts with Cas9 or Cas12. Invariant guide RNA hairpin sequences can be provided based on common knowledge in the art, for example, as disclosed by Nishimasu et al. (Nishimasu H, et al. Crystal structure of Cas9 in complex with guide RNA and target DNA. Cell. 2014; 156: 935-949). Any invariant hairpin sequence can be used, as long as it can bind to Cas nuclease post-transcriptionally.

In some embodiments, the sgRNA from 5' to 3' comprises: a guide sequence and a repeat-anti-repeat stem-loop. In some embodiments, the sgRNA from 5' to 3' comprises: a guide sequence, a repeat-anti-repeat stem loop, and stem loop 1, stem loop 2 and/or stem loop 3.

In addition, this application also provides a CRISPR system comprising the above-mentioned gRNA or its construct, and a CAS enzyme or its encoded nucleic acid or construct. In some embodiments, the CRISPR system is a CRISPR/Cas9 system. In some embodiments, the CRISPR system is a CRISPR/Cas12 system.

## EXAMPLE

The following examples and exemplary embodiments are intended to be purely illustrative of this application and should not be construed as limiting this application in any way. The following examples and detailed descriptions are provided in an illustrative manner rather than a limiting one.

Furthermore, as used in the following examples, the abbreviations for cell treatment and their corresponding meanings are as follows:

TSKO: Double knockout of TCR and SPPL3;
TKO: TCR knockout;
TBKO: Double knockout of TCR and B2M genes;
TFKO: Double knockout of TCR and Fas genes;
TBSKO: Triple knockout of TCR, B2M and SPPL3 genes.

### Example 1: Preparation of U-CART cells

### 1. Isolation and activation of T cells from healthy donor

Collection of cord blood from healthy donors: After obtaining cord blood from the blood bank, it was transported to a GMP laboratory via a cold chain logistics vehicle equipped with temperature control equipment for T cell isolation.

1.1 Preparation of peripheral blood mononuclear cells

Physiological saline was aspirated with a pipette and added to the umbilical cord blood delivered to the laboratory, and the umbilical cord blood was diluted with the physiological saline at a ratio of 1:1 (V/V). The blood cell diluent was slowly added to a lymphocyte separation tube, followed by centrifugation at 800×g for 20 minutes. The buffy coat cells above the lymphocyte separation medium were aspirated and transferred into a new 50 mL centrifuge tube, and T cell medium (Miltenyi Biotec, Cat. No. 170-076-306) was added thereto. After centrifugation at 400×g for 5 minutes, the supernatant was discarded, and the cell pellet at the bottom of the centrifuge tube was retained, whereby peripheral blood mononuclear cells were obtained.

1.1.2 Isolation and activation of T cells

The obtained peripheral blood mononuclear cells were counted using a cell counter (Nexcelom, model: Cellometer K2) and then sorted for T cells. The specific steps were as follows: The cell pellet was adjusted to a density of $5*10^7$/ml using Easy buffer (manufacturer: StemCell, catalog number: 16F72331), and the cells were transferred to a 5ml flow cytometer using a 5ml pipette; T cell separation reagent was added at a concentration of 50μl/ml, and incubated at room temperature for 5 minutes after addition; sorting magnetic beads were added, and the beads were mixed within 30 seconds, with a concentration of 40μl/ml; the cell suspension was brought up to 2.5ml using Easy buffer, and the tube was placed directly on the magnetic column for 3 minutes. Then the cells were poured into a 15ml centrifuge tube, which yielded T cells; after sorting, the cells were mixed with 1000μl pipette, counted, centrifuged (400×g, 5min), and the supernatant was discarded to obtain the T cell pellet. The T cell pellet was resuspended in the T cell culture medium. Then, T cell activating factors (magnetic beads coated with anti-CD3/CD28 antibodies) were added at a 1:1 ratio. At this point, the T cells were in an activated state. The T cells were then placed in a 37°C, 5%$CO_2$ incubator for further expansion and culture. CAR molecules were transduced into activated T cells using lentiviruses. The CAR molecule used in the example of this application is a CAR that specifically binds to CD19, and the extracellular antigen-binding domain used is the scFV of the anti-CD19 monoclonal antibody FMC63. Its hinge region and transmembrane region are the hinge region and transmembrane region of the CD8α molecule, respectively. The intracellular signaling domain is composed of a 4-1BB co-stimulatory domain and CD3Z connected in tandem.

**1.2 Gene knockout of T cells**

The TRAC, B2M, and SPPL3 genes in the T cells obtained in step 1.1.2 were knocked out using CRISPR/Cas9 gene editing technology. The specific steps were as follows:
1.2.1 Design of sgRNA and construction of plasmids targeting the α-strand constant coding region (TRAC) gene of TCR, the B2M gene of HLA constant coding region, and the constant coding region gene of SPPL3.
All sgRNAs designed targeting the coding sequences of each exon in the TRAC, B2M, and SPPL3 coding regions were designed using CRISPR RGEN Tools. For the CRISPR/Cas9 system, the sequences targeted by the sgRNA regions are listed in the sequence listing at the end of this specification as SEQ ID NO:1-256, wherein SEQ ID NO:68, SEQ ID NO:145, SEQ ID NO:150, SEQ ID NO:154, SEQ ID NO:155, SEQ ID NO:159, SEQ ID NO:161, SEQ ID NO:165, SEQ ID NO:172, SEQ ID NO:176, SEQ ID NO:196, SEQ ID NO:203, SEQ ID NO:204, SEQ ID NO:209, SEQ ID NO:210, SEQ ID NO:212, SEQ ID NO:241, and SEQ ID NO:250 represent sgRNAs targeting complementary sequences of the SPPL3 genomic region. SEQ ID NO:1 and SEQ ID NO:2 represent sgRNAs targeting complementary sequences of the TRAC genome region. For the CRISPR/Cas12 system, the sequences of the sgRNA targeting regions are listed in the sequence listing at the end of the specification as SEQ ID NO:257-316, wherein SEQ ID NO:261, SEQ ID NO:262, SEQ ID NO:263, and SEQ ID NO:264 represent sgRNAs targeting complementary sequences of the SPPL3 genome region, and SEQ ID NO:257, SEQ ID NO:258, SEQ ID NO:259, and SEQ ID NO:260 represent sgRNAs targeting complementary sequences of the TRAC genome region.
1.2.2 sgRNA was subjected to 2'-O-methylation and/or internucleotide thiophosphate modification by chemical methods to obtain sgRNA with high knockout efficiency and stability.
1.2.3 Cas9 plasmid and GFP plasmid, XbaI (manufacturer: NEB, catalog number: cat#R0145S), and cutsmart buffer (manufacturer: NEB, catalog number: cat#B7204s) was taken for restriction enzyme digestion and linearization. The 50 μl reaction system was shown in Table 1.

Table 1:

| Component | Volume |
|---|---|
| Cas9 plasmid | 5μg |
| 10*cutsmart Buffer | 5μl |
| Xbal | 2μl |
| water was added to a total reaction volume | 5μl |

After incubating in a 37°C water bath for 4 hours, 2 μl of the enzyme digestion product was taken for agarose gel electrophoresis. The voltage was set to 110 U, and the electrophoresis was performed for 30 min. A single band was observed under the gel imaging instrument indicating that the enzyme digestion was complete and all plasmids were linearized.

The above reaction product was taken, cleaned and purified.

The purified product was used for in vitro transcription (IVT) using the HISCRIBE™ T7 ARCA mRNA Kit (manufacturer: NEB, catalog number: cat#E2060S). The 20 μl system was shown in Table 2.

Table 2:

| Component | Volume |
|---|---|
| 2*ARCA Mix | 10μl |
| Template DNA | 0.5μg |
| T7 RNA Polymerase Mix | 2μl |
| Nuclease-free water | To 20μl |
| Total reaction volume | 20μl |

The above reaction system was placed on a PCR instrument and reacted at 37°C for 4 hours. Four hours later, 2 μl of DNase 1 was added to the reaction system, and the reaction was carried out at 37°C for 20 min.

The above reaction products were taken, and the operations shown in Table 3 was performed.

Table 3:

| Component | Volume |
|---|---|
| Nuclease-free water | 63μl |
| IVT reaction | 22μl |
| 10*Poly(A) polymerase buffer | 10μl |
| Poly(A) polymerase | 5μl |
| Total reaction volume | 100μl |

The above reaction system was placed on a PCR instrument and reacted for 2 hours. The above reaction products were cleaned and purified, and stored in a -80°C freezer for later use.

1.2.4 T cells cultured by Step 1.1.2 were collected into a 50 mL centrifuge tube and centrifuged at $300\times g$ for 7 minutes, after which the supernatant was discarded. The cells were washed twice with DPBS solution (Manufacturer: Gibco, Cat. No.: 1924294), and then the cell density was adjusted to $2.5\times10^7$ cells/mL using an electroporation reagent. Cas9 mRNA and sgRNAs were mixed with the T cells to reach a final concentration of $2.5\times10^6$ cells and 8 μg of total RNA per 100 μL (2 μg for each of Cas9 mRNA, TCR sgRNA, B2M sgRNA and SPPL3 sgRNA). Subsequently, Cas9 mRNA and sgRNAs were introduced into the cells using an electroporator BTX Agile Pulse MAX (Manufacturer: BTX, Model No.: 47-0200NINT) under the electroporation conditions of 200-400V/0.5-2ms. Based on the antigen screening principle, T cells that were negative for TCR and/or B2M and/or SPPL3 and simultaneously positive for CD4 and CD8 were screened out, whereby U-CAR-T cells were obtained.

The cells were used to extract the genome using the TIANamp Genomic DNA Kit (manufacturer: TIAN GEN, catalog number: cat#DP304-03). Using synthetic primers, the extracted cell genome was taken and PCR amplified with 2*Esay Taq Super Mix (+dye) (manufacturer: TRANS, catalog number Code#AS111) to amplify the genomic regions comprising the corresponding sgRNAs of TRAC, B2M and SPPL3, respectively. The reaction system was 50 μl, as shown in Table 4.

Table 4:

| Component | Volume |
|---|---|
| 2*Easy Taq Super Mix(+dye) | 25μl |
| Primer F (10 pmol/μl) | 1μl |
| Primer R (10 pmol/μl) | 1μl |
| Template DNA | 300ng |
| Water was added to a total volume | 50μl |

The reaction conditions are as follows:

95°C 3min

95°C 30s

Variable 30s 72°C ⎫ 35 cycles

1.2.5 The PCR products after the reaction were subjected to NGS sequencing to verify the knockout efficiency of TCR, HLA, and SPPL3 at the molecular level. After 8 days of cell culture, the resulting T cells were subjected to quality control monitoring. The results show that the efficiency of knocking out TCR alone is about 97.18%, and the efficiency of knocking out SPPL3 is about 96.98%. It is evident that, under this system, CRISPR/Cas9 technology can successfully edit the constant coding sequences of TCR's TRAC gene, HLA's B2M gene, and SPPL3, including insertion and deletion mutations, all of which result in frameshift mutations, thereby suppressing the expression of TCR, HLA, and SPPL3 at the gene level.

1.2.6 Detection of off-target rates of TCR and SPPL3 genes in U-CART cells

Simultaneously, potential off-target sites on the human genome for TRAC (SEQ ID NO: 1 and 2) and SPPL3 (SEQ ID NO: 155 and 166) were predicted, and the predicted off-target site regions that might affect the expression of other genes were amplified and analyzed. The aim was to confirm at the molecular level that the knockout of TRAC and SPPL3 did not introduce the knockout of non-specific off-target genes. The results show that the TRAC and SPPL3 genes are completely knocked out in U-CART cells obtained after gene editing of T cells using sgRNAs targeting TRAC (including any one of SEQ ID NO: 1-4) and sgRNAs targeting SPPL3 (including any one of SEQ ID NO: 6-256) and then screening them. At the same time, no gene mutations are found at potential off-target sites. The specific results are shown in Tables 5 and 6.

Table 5: Off-target analysis results of TRAC knockout T cells

| sgRNA | Potential off-target sites | INDEL (%) |
|---|---|---|
| SEQ ID NO:1 and SEQ ID NO:2 | chrX:73096741-73096783 | 0 |
| | chr15:45003596-45003638 | 0 |
| | chr7:97861900-97861942 | 0 |
| | chr2:161504884-161504926 | 0 |
| | chr7:147683672-147683714 | 0 |
| | chr18:42654204-42654246 | 0 |
| | chr8:10627233-10627275 chr1:151805238-151805280 | 0 0 |
| | chr16:85219576-85219618 | 0 |
| | chr10:31873265-31873307 | 0 |

(continued)

| sgRNA | Potential off-target sites | INDEL (%) |
|---|---|---|
| | chr12:122582230-122582272 | 0 |
| | chr10:134549611-134549653 | 0 |
| | chr22:21970051-21970093 | 0 |
| | chr14:23016448-23016490 | 81 |

Table 6: Off-target analysis results of SPPL3 knockout T cells

| sgRNA | Potential off-target sites | INDEL (%) |
|---|---|---|
| SEQ ID NO:155 and SEQ ID NO:166 | chrX:20050414-20050635 | 0 |
| | chr20:61460399-61460610 | 0 |
| | chr20:12918718-12918920 | 0 |
| | chr19:6419231-6419427 | 0 |
| | chr18:21811933-21812124 | 0 |
| | chr17:80094136-80094357 | 0 |
| | chr22:35439798-35439985 | 0 |
| | chr22:28288021-28288239 | 0 |
| | chr22:23799869-23800085 | 0 |
| | chr20:61466737-61466938 | 0 |
| | chr20:61067848-61068074 | 0 |
| | chr20:16284793-16285003 | 0 |

1.2.7 Determination of relevant viability indicators of U-CART cells constructed using CRISPR/Cas9 and CRISPR/ Cas12 systems before purification

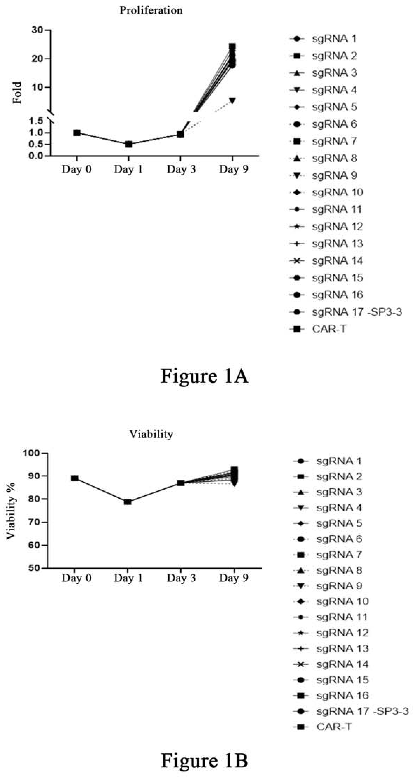

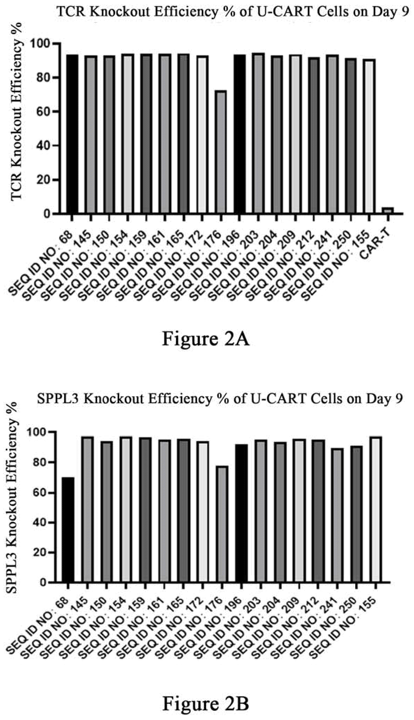

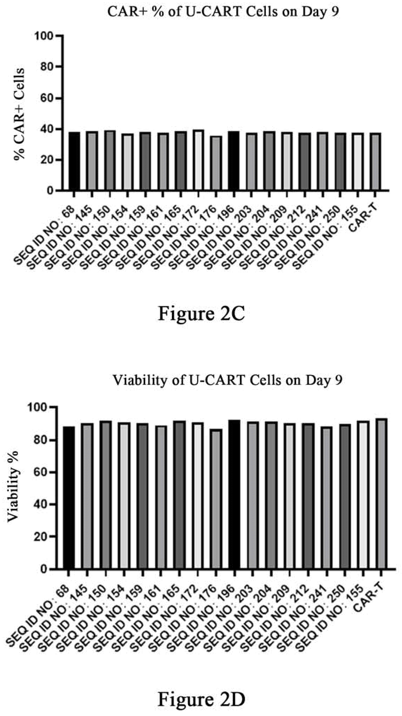

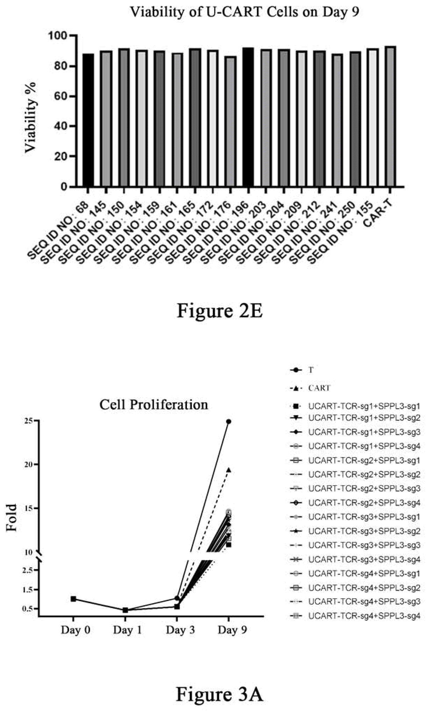

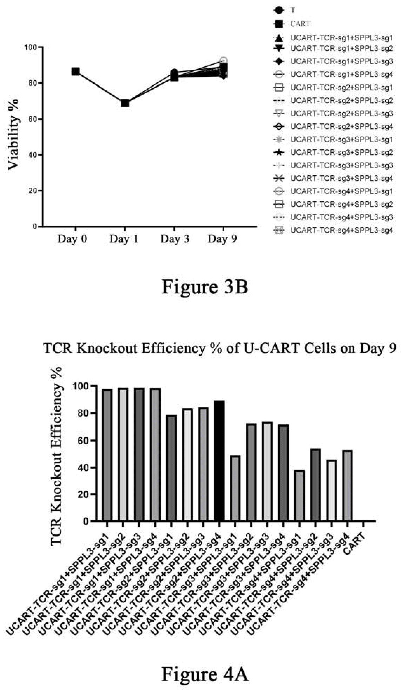

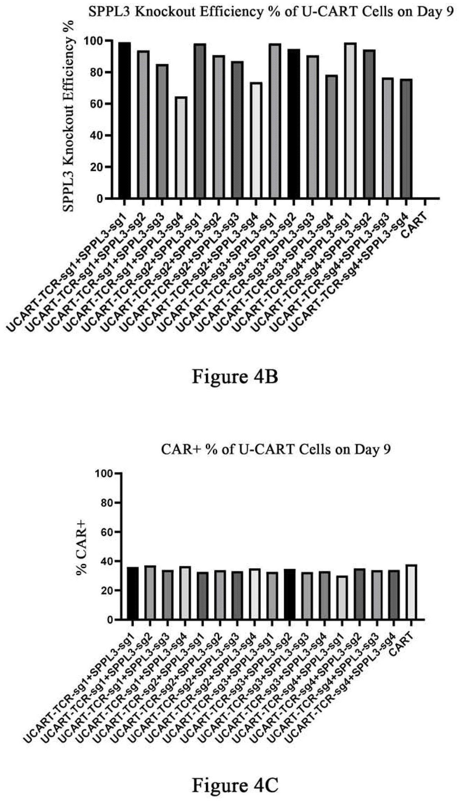

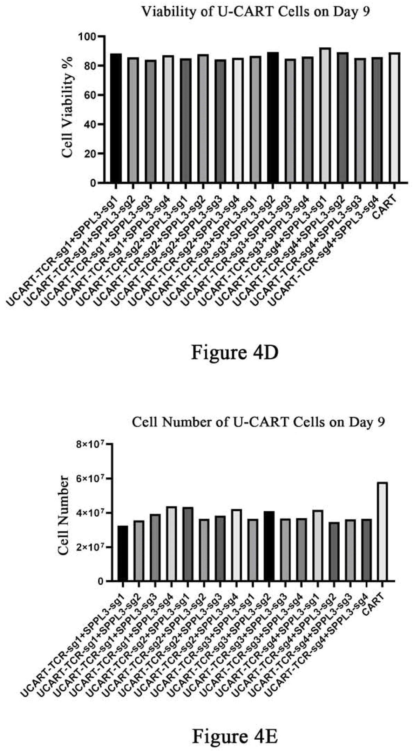

For the CRISPR/Cas9 system, 17 sgRNAs (SEQ ID NO:68, SEQ ID NO:145, SEQ ID NO:150, SEQ ID NO:154, SEQ ID NO:155, SEQ ID NO:159, SEQ ID NO:161, SEQ ID NO:165, SEQ ID NO:172, SEQ ID NO:176, SEQ ID NO:196, SEQ ID NO:203, SEQ ID NO:204, SEQ ID NO:209, SEQ ID NO:212, SEQ ID NO:241, SEQ ID NO:250) targeting the complementary sequence of the SPPL3 genomic region and 1 sgRNA (SEQ ID NO:1) targeting the complementary sequence of the TCR genomic region were combined to double knock out SPPL3 and TCR. The proliferation, viability, TCR KO efficiency on day 9, and SPPL3 KO efficiency on day 9, CAR+ cell percentage on day 9, U-CART cell viability on day 9, and U-CART cell number on day 9 of the obtained U-CART cells were measured, and the results are shown in Figures 1A-2E. For the CRISPR/Cas12 system, 4 sgRNAs (SEQ ID NO:261, SEQ ID NO:262, SEQ ID NO:263, SEQ ID NO:264) targeting the complementary sequence of the SPPL3 genomic region and 4 sgRNAs (SEQ ID NO:257, SEQ ID NO:258, SEQ ID NO:259, SEQ ID NO:260) targeting the complementary sequence of the TCR genomic region were combined to double knock out SPPL3 and TCR. The proliferation, viability, TCR KO efficiency on day 9, SPPL3 KO efficiency on day 9, CAR+ cell percentage on day 9, U-CART cell viability on day 9, and U-CART cell number on day 9 of the obtained U-CART cells were measured, and the results are shown in Figures 3A-4E.

The results above demonstrate that the U-CARTs edited using both the CRISPR/Cas9 and CRISPR/Cas 12 systems achieve the expected experimental results and can be further purified.

1.3 U-CART cells prepared by double knockout of TCR and SPPL3 genes were purified according to the following experimental steps:

U-CART cells were washed with 1% HSA buffer and then resuspended at a density of 1E8/mL. TCRα/β-Biotin was added at a dosage of 100 μL per mL, and the cells were placed on a shaker at 25 rpm for incubation at room temperature for 30 min. The cells were washed twice with buffer containing 1% HSA, then resuspended in buffer containing 1% HSA. Anti-Biotin Reagent was added at a dosage of 200 μL per mL, and the cells were placed on a shaker at 25 rpm for incubation at room temperature for 30 min. The cells were washed once with buffer containing 1% HSA and resuspended in buffer containing 1% HSA. The cells were passed through an LS Column (Miltenyi Biotec, Cat. No.: 130-042-401), the negative cells were collected for viable cell counting, and the purification efficiency was detected by flow cytometry.

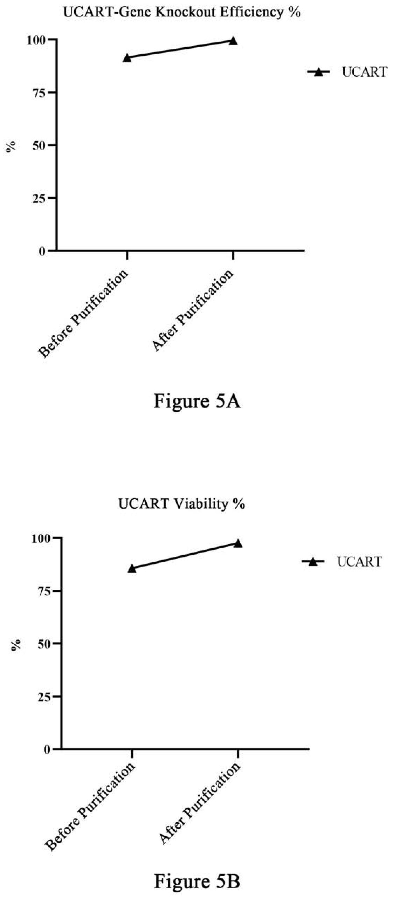

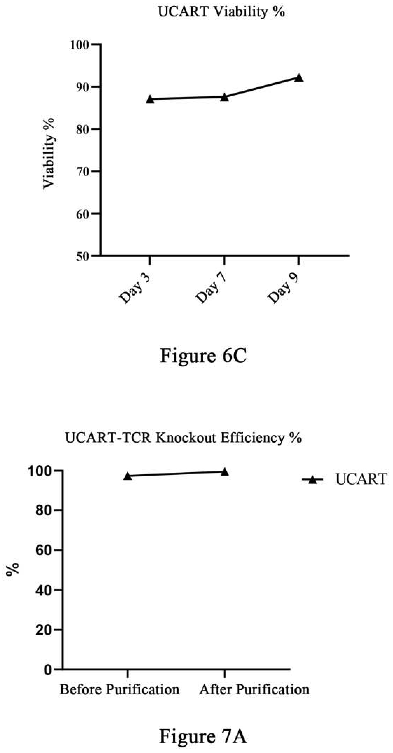

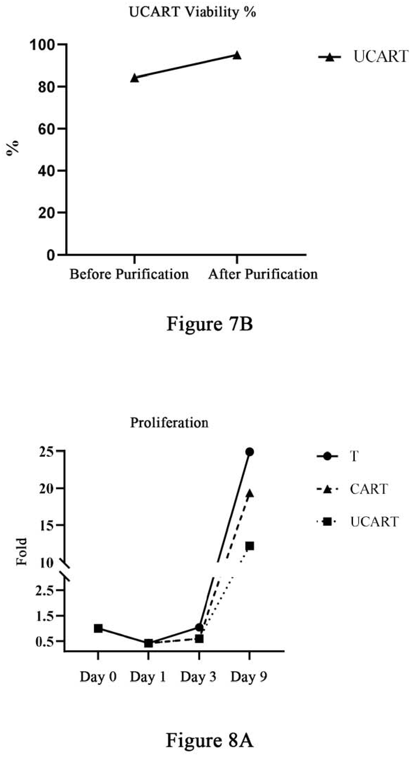

The results, as shown in Figures 5A-5B and 7A-7B, indicate that the viability of U-CART cells do not decrease after purification.

**Example 2: Functional Verification of U-CART**

### 2.1 Proliferation and Viability Detection

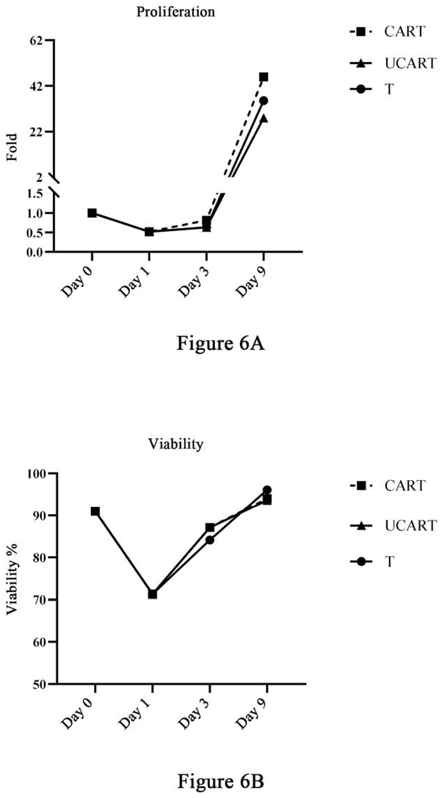

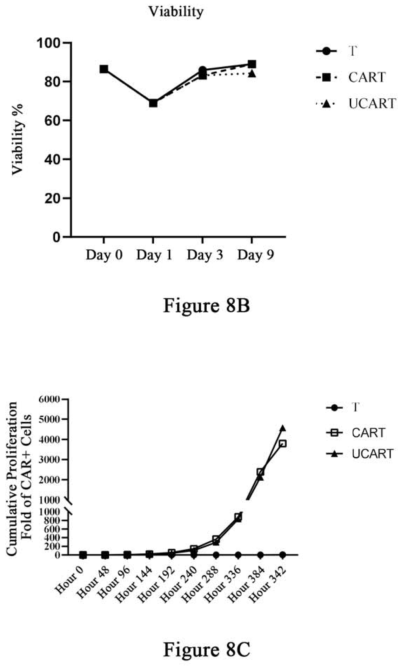

U-CART cells (U-CART) with complete knockout of TRAC and SPPL3 genes prepared in Example 1, unmodified T cells (T), and ordinary CAR-T cells (CART) without any gene knockout were tested for proliferation ability and cell viability under completely identical conditions. The results are shown in Figures 6A-6B and 8A-8B. It can be seen that knocking out TCR and SPPL3 genes does not affect the proliferation and cell viability of U-CART cells. Figures 6C and 8C show that after knocking out the TCR and SPPL3 genes, the cell viability of U-CART cells does not decrease with the expansion of U-CART cells.

### 2.2 Detection of the ability to resist allogeneic T cell killing

To assess whether SPPL3-knockout U-CART cells would be cleared by T cells in patients after infusion, the 1G4-NY-ESO-1 system (Wei Wensheng Laboratory, Peking University) was used as an experimental model. The experimental system uses T cells expressing 1G4 TCR, which specifically kill NY-ESO-1 positive HLA-A 02-01 subtype T cells. After the B2M gene is knocked out, NY-ESO-1 positive HLA-A 02-01 subtype T cells cannot present the NY-ESO-1 antigen peptide to the cell surface, thus resisting the killing effect of 1G4 TCR-T cells.

The specific experimental protocol was as follows: the donor's 1G4 TCR-T cells were resuspended in AIM5+5% FBS medium and the cell concentration was adjusted to $5*10^6$/mL. NY-ESO-1 positive HLA-A 02-01 subtype T cells after different gene editing were resuspended in AIM5+5% FBS medium, and the cell concentration of each group was adjusted to $1*10^6$/mL. 1G4 TCR-T cells and NY-ESO-1 T cells were seeded into 96-well U-shaped plates at an effector-target ratio (1G4:NY-ESO-1 = 5:1). 100 $\mu$L of 1G4 TCR-T and NY-ESO-1 T cells were added to each well, for a total of 200 $\mu$L per well. After incubation for 24 hours, 20 $\mu$L of cells were taken from each well for K2 counting to calculate the total number of viable cells. The remaining 180 $\mu$L was used to detect the ratio of 1G4 TCR-T and NY-ESO-1 positive T cells using flow cytometry (Note: 1G4 TCR-T cells are GFP-tagged and FITC-positive detected by flow cytometry; NY-ESO-1 positive T cells are BFP-tagged and PB450-positive detected by flow cytometry). The killing rate of NY-ESO-1 positive T cells in each experimental group was calculated by comparing the total number of $1*10^5$ NY-ESO-1 positive T cells at the time of plating.

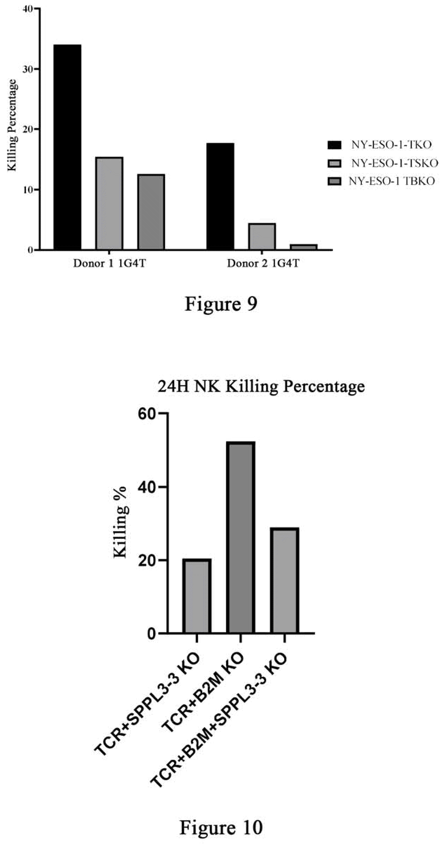

The results, as shown in Figure 9, indicate that SPPL3 knockout significantly enhances the ability of T cells to resist allogeneic T cell killing. In terms of enhancing the ability of T cells to resist the killing effect of allogeneic T cells, knocking out SPPL3 can achieve the same or slightly weaker effect than knocking out B2M.

### 3. Detection of the ability to resist NK cell killing

NK cells and U-CART cells were plated at a ratio of NK:T = 10:1, with 2E5 U-CART cells per group. The cells were cultured in AIM-V + 5% FBS medium and incubated overnight at 37°C. The sample was collected 24 hours later. After blowing evenly, 200 $\mu$L of cells were taken from each well for flow cytometry analysis to determine the killing ratio. Cell killing was detected using the FACS counting method.

The results are shown in Figure 10, wherein the percentage of cell killing =(number of U-CART cells in control wells - number of U-CART cells in interaction wells) / number of U-CART cells in control wells * 100%.

The results show that B2M knockout lead to enhanced killing effect of allogeneic NK cells; knocking out SPPL3 while knocking out B2M lead to a relative reduction in the killing effect of NK cells; knocking out SPPL3 without knocking out B2M at all significantly reduce the killing effect of allogeneic NK cells, thereby enhancing the persistence of U-CART cells in patients.

### 4. Detection of the ability to resist allogeneic PBMC killing

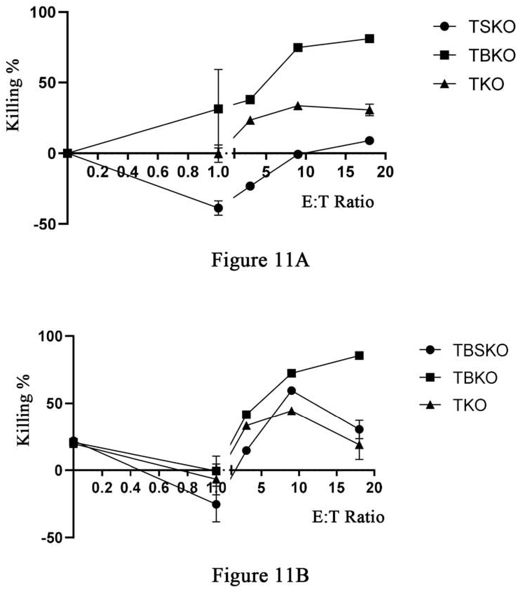

To evaluate the effect of SPPL3-knockout U-CART cells on the combined killing effect of allogeneic T cells and NK cells after infusion into patients, U-CART cells were co-incubated with allogeneic PBMCs, and the killing effect of allogeneic PBMCs on U-CART cells was statistically analyzed. The specific experimental protocol was as follows: (1) First, three types of U-CART cells, namely U-CAR-T cells with TCR and SPPL3 knocked out (TSKO), U-CART cells with TCR knocked out only (TKO), and U-CART cells with TCR and B2M knocked out (TBKO) were subjected to CellTrace staining (Figure 11A, TKO-CellTrace-CFSE, TBKO-CellTrace-Far Red, TSKO-CellTrace-VIOLET); or (2) First, three types of U-CART cells, namely U-CAR-T cells with TCR, B2M and SPPL3 knocked out (TBSKO), U-CART cells with TCR knocked out only (TKO), and U-CART cells with TCR and B2M knocked out (TBKO) were subjected to CellTrace staining (Figure 11B, TKO-CellTrace-CFSE, TBKO-CellTrace-Far Red, TSKO-CellTrace-VIOLET); then the three types of U-CART cells were mixed in a 1:1:1 ratio. The mixed U-CART cells and allogeneic PBMCs were incubated in an incubator at 37 °C with 5% $CO_2$ at different ratios (the ratio of PBMCs to U-CART cells, represented by E:T in the figure). After 120 hours, the number of viable cells of TKO, TBKO, and TSKO/TBSKO was counted by flow cytometry absolute counting. Compared with the control group without allogeneic PBMCs, the killing percentage of allogeneic PBMCs on the four types of U-CART cells was calculated according to the formula: killing percentage = (number of viable cells in the control group - number of viable cells in the experimental group) / number of viable cells in the control group * 100%.

The results, as shown in Figures 11A and 11B, indicate that when using PBMC cells for cell killing, knocking out TCR and SPPL3 (TSKO) significantly reduces the killing effect of PBMCs on U-CART cells compared to knocking out TCR alone (TKO). However, further knocking out B2M (TBKO) on the basis of knocking out TCR enhances the killing effect of PBMCs on U-CART cells. Furthermore, knocking out SPPL3 (TBSKO) on the basis of knocking out TCR and B2M can also partially weaken the killing effect of PBMCs on U-CART cells.

It is evident that SPPL3 knockout in the PBMC environment or in vivo environment can reduce the killing effect of the body's immune cells on allogeneic T cells with normal HLA-I expression (such as TSKO cells in this example) and T cells with HLA-I expression eliminated or suppressed (B2M knockout).

### 5. Detection of the ability to resist killing mediated by FasL

The experimental protocol for this application was as follows:

1. CAR-T cells of TKO, TFKO and TSKO were taken, with a total cell number of $1 \times 10^7$ cells for each subtype; the cells were resuspended in 10 mL of buffer, and plated in a 24-well plate, with 1 mL of the cell suspension added to each well.
2. The concentration of Biolegend Recombinant Human FASL was adjusted to 1000 $\mu$g/mL using T cell complete culture medium, as a stock solution.
3. The FASL stock solution was diluted with T cell complete culture medium to 500, 200, 100, and 50 $\mu$g/mL.
4. The FASL was added to 24-well plates comprising cells at a volume ratio of 1:1000, so that the final concentrations of FASL in each group were 1000, 500, 200, 100, 50, and 0 ng/mL.
5. The 24-well plate was placed in an incubator at 37 °C with 5% $CO_2$ and incubated for 24 hours.
6. After 24 hours, the 24-well plate was taken out, and the cells were pipetted to homogeneity with a 1 mL pipette. 20 $\mu$L of AOPI dye was added to 20 $\mu$L of the cell suspension, and the cell viability of each group of cells was counted using a K2 Counter, with the counting results recorded.

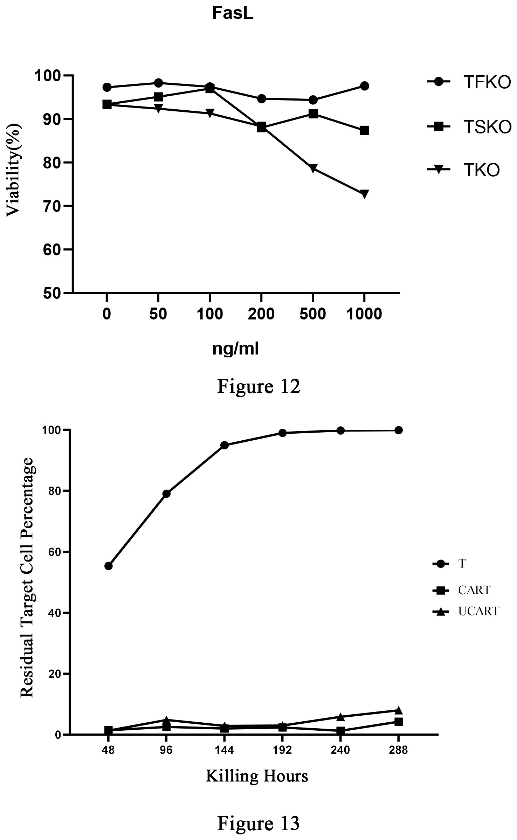

The results, as shown in Figure 12, indicate that knocking out SPPL3 can partially achieve the effect of knocking out FasL, that is, it can resist the apoptosis of U-CART cells induced by the Fas signaling pathway.

### 6. Detection of the in vitro killing ability of U-CAR-T cells

Validation was performed using the U-CART cells with complete knockout of TRAC and SPPL3 genes prepared in Example 1 (U-CART), CAR-T cells without gene knockout (CART) and unmodified T cells as effector cells, and Nalm6 (human acute B-lymphoblastic leukemia cell line) as the target cell. The specific method was as follows:

Cell resuscitation: Cryopreserved U-CART cells, T cells and target Nalm6 cells (human acute B-lymphoblastic leukemia cell line) were rapidly thawed in a water bath at 37°C. When almost no ice crystals remained in the cryopreservation tube, the cells were transferred to a 15 mL centrifuge tube and centrifuged at 400 $\times$ g for 5 min for collection, then resuspended in fresh medium and counted.

Co-incubation of target cells and effector cells: The aforementioned effector cells and target cells were co-added to cell culture plates at an effector-to-target (E:T) ratio of 1:2, respectively. The culture plates were then transferred to a cell incubator and incubated at 37°C with 5% $CO_2$.

Sustained addition of target cells: Cells were collected for counting and flow cytometry analysis every 48 hours, and the proportions of target cells and CAR-positive (CAR$^+$) cells were statistically determined. Based on the CAR$^+$ percentage and total cell number of each cell population, effector cells and target cells were again co-added to cell culture plates at an E:T ratio of 1:2, respectively.

The above operations were repeated until the remaining number of CAR$^+$ cells were insufficient for the continuation of the co-incubation experiment.

The following information was collected and the relevant parameters were calculated after each co-incubation:
Residual percentage of target cells: the proportion of CD4$^-$CD8$^-$ cells was statistically determined, namely the percentage of target cells in the total cell population;

Total number of CAR$^+$ cells: total CAR$^+$ cell number = CAR$^+$ percentage $\times$ total sample cell number;

Cumulative proliferation fold of CAR$^+$ cells = (current total CAR$^+$ cell number / total CAR$^+$ cell number at the last plating) $\times$ cumulative proliferation fold of CAR$^+$ cells at the last plating (the cumulative proliferation fold of CAR$^+$ cells at 0 h was defined as 1).

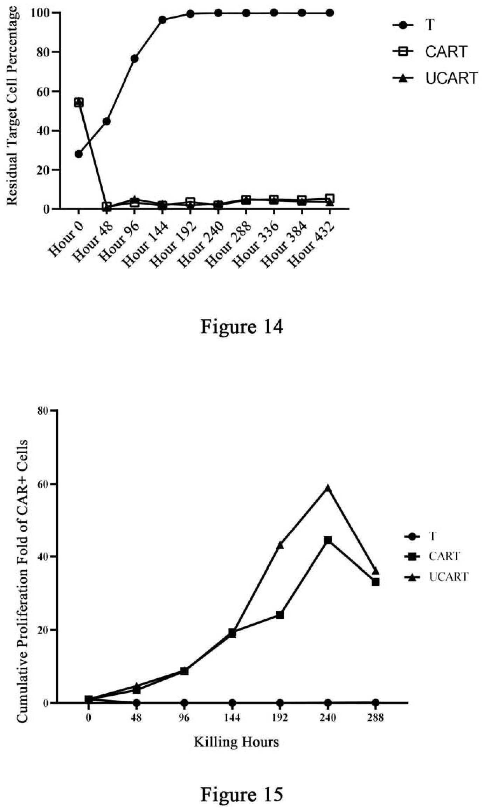

The test results of the killing ability of different T cells and the proliferation of CAR[+] T cells are shown in Figures 13-14 and 15. As shown in Figures 13-14, the killing effects of CAR T and U-CART cells are superior to those of T cells, and although U-CART cells are subjected to gene editing, no statistically significant difference is observed in their killing ability against target cells compared with CAR T cells in the normal control group. Analysis of the residual proportion of target cells indicates that U-CART cells exert a significant killing effect on Nalm6 target cells, and the residual proportion of target cells induced by the killing effect of U-CART cells remains below 10% throughout the long-term killing experiment. Figure 15 shows that T cells without CAR molecule transduction exhibit no CAR[+] cell proliferation and no killing or inhibitory effect on target cells, which results in the increase in the proportion of target cells with time as shown in Figure 8; the proportion of target cells in the total cell population exceeds 90% 144 h after co-incubation. In contrast, the proliferation of CAR[+] cells in U-CART cells is maintained for up to 288 h after co-incubation as shown in Figure 15, and the peak proliferation fold is basically consistent with that of CAR-T cells. In summary, U-CART cells exhibit robust expansion upon stimulation with target cells and exert a long-term and significant killing and inhibitory effect on tumor cells.

### 7. Detection of the in vivo killing ability of U-CART cells

In this example, highly immunodeficient NCG mice were inoculated with human acute B-lymphoblastic leukemia cells (Nalm-6) to establish a tumor-bearing model. Control T cells, CAR-T cells, and U-CART cells prepared from the same healthy donors were then injected to evaluate the tumor-suppressing effect of U-CART cells in tumor-bearing mice.

Twenty female NCG mice aged 6-8 weeks were used in the experiment, and a model was established via tail vein injection of Nalm6-LAE cells (Nalm6-Luciferase-LAE) at a dose of $1\times10^6$ cells per mouse. On the 4th day after tumor cell inoculation, in vivo imaging was performed to detect tumor burden, and the mice were randomly divided into four groups according to the fluorescent values from imaging. CAR-T cells ($6\times10^6$ CAR[+] cells per mouse) and U-CART 2.0 cells ($6\times10^6$ CAR[+] cells per mouse) were administered to the corresponding groups, with 5 mice in each group; unedited T cells or U-CART cell cryopreservation solution derived from the same volunteer were concomitantly administered to the other two groups, with 5 mice in each group, which were designated as the T cell control group and the tumor-bearing control group, respectively. All animals were administered via a single tail vein injection, and the administration day was defined as Day 1. After administration, in vivo imaging was performed once a week to detect tumor burden; the survival status of the mice was observed and recorded daily; the body weights of the animals were measured and recorded twice a week. If a mouse exhibited a body weight loss of more than 20% during the experiment, the animal will be recorded as dead and euthanized.

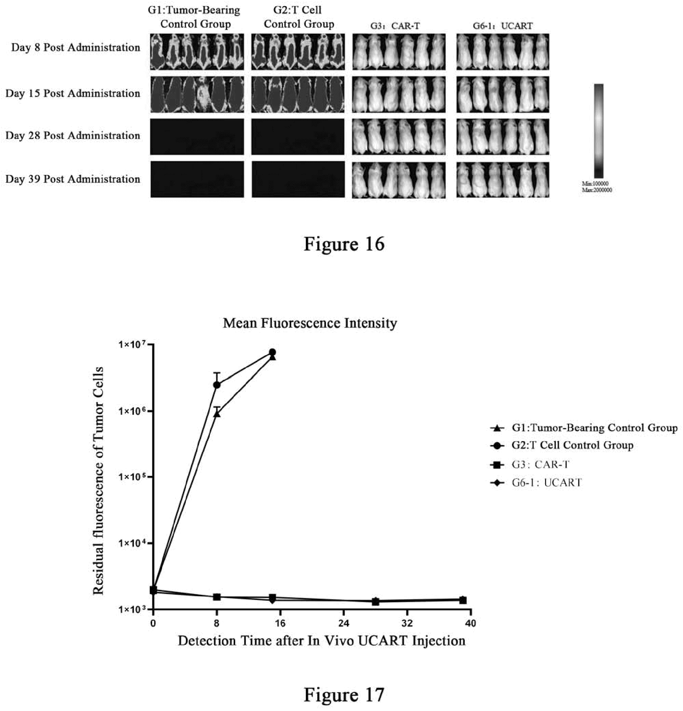

The results are shown in Figure 17, and it can be seen from the in vivo imaging results (Figure 16) that after the injection of CAR-T and U-CART cells, the tumor growth rate in the tumor-bearing control group and the T cell control group is faster than that in the test groups. In addition, the tumor growth rate in the T cell control group is consistent with that in the tumor-bearing control group, indicating that T cells themselves have no tumor cell killing function. Compared with the CAR-T cell group, no tumor recurrence is observed in the animals of the U-CART cell group until day 39 after administration. The U-CART cells with double knockout of TCR and SPPL3 genes exhibit an extremely potent tumor cell killing ability in immunodeficient mice, which is not inferior to that of the unedited CAR-T cells. It can be seen that gene editing does not impair the in vivo killing ability of CAR-T cells nor affect their long-term in vivo survival.

The sequences used in the above examples of the present application are shown in the sequence listing below. It should be understood that the following sequences are merely exemplary sequences for the embodiments of the present application and do not constitute any limitation to the solutions of the present application. The nucleic acid sequences in the following sequence listing may represent DNA sequences or RNA sequences; when representing RNA sequences, the "T" therein denotes uridine.

### Sequence Listing:

| SEQ ID NO. | Target | Sequence |
|---|---|---|
| For the CRISPR/Cas9 system | | |
| 1 | | GCTGGTACACGGCAGGGTCA |
| 2 | Targeting TRAC | CTCTCAGCTGGTACACGGCA |
| 3 | | ATTTGTTTGAGAATCAAAAT |
| 4 | | TCTCTCAGCTGGTACACGGC |

(continued)

| SEQ ID NO. | Target | Sequence |
|---|---|---|
| For the CRISPR/Cas9 system | | |
| 5 | Targeting B2M | GAGTAGCGCGAGCACAGCTA |
| 6 | | AGCAAGCAAGCACCGGACCC |
| 7 | | GGCGGCGGCGCGGAGAACAA |
| 8 | | GCCATGGCGCTGCCTCTCGT |
| 9 | | CACTCGATCACACCCGCCGA |
| 10 | | GGGGCGGGCGAACGGCAAGA |
| 11 | | CGGCGGCGGCGGGCTCGCTC |
| 12 | | CCTGCAGCGGAGCCCACGAG |
| 13 | | GGGCGGGCGAACGGCAAGAC |
| 14 | | TCTTGCCGTTCGCCCGCCCC |
| 15 | | GCGGCGGCGCGGAGAACAAG |
| 16 | | GCCCCGCGCGCAGTGAGCGC |
| 17 | | CTGGCTCGCTGGCTGCACGG |
| 18 | | CGGCGGCGCGGAGAACAAGG |
| 19 | Targeting gRNA of SPPL3 exon 1 | CCACTCGATCACACCCGCCG |
| 20 | | CCCGGCCGCGCCTTCAGCTA |
| 21 | | CTCTCGGCCTCCCTCACCCG |
| 22 | | GAGGGGGGCGCTAGGCGATG |
| 23 | | GGAGAGGCCGGGCTCCGAAG |
| 24 | | CCGAGCGAGCCCGCCGCCGC |
| 25 | | CCTCGGCGGGTGTGATCGAG |
| 26 | | GGCCCGCGCTCACTGCGCGC |
| 27 | | GCGGGAGCGCCGCGCTCGGG |
| 28 | | CGGAGGCGGCGGCGACTGAG |
| 29 | | CTCGCTCGGGCCGTAGCTGA |
| 30 | | CCCAGGGAGCGGGGCCGCTT |
| 31 | | CGGCGGCGGCGCGGAGAACA |
| 32 | | TCGGCCTCCCTCACCCGCGG |
| 33 | | TGGCTCGCTGGCTGCACGGC |
| 34 | | CCGAAGCGGCCCCGCTCCCT |
| 35 | | CGCCCGCCGGCCATGTCCCC |
| 36 | | GCGGAGGCGGCGGCGACTGA |
| 37 | | TCGGGCCGTAGCTGAAGGCG |
| 38 | | CTCCAAACCCAACATGGCGG |
| 39 | | CTTGCCGTTCGCCCGCCCCA |
| 40 | | CCGCCGCCGCCGCCCGAGCG |
| 41 | | TGCACGGCGGGCCGGGAAGG |
| 42 | | GGAGCGCCGCGCTCGGGCGG |

(continued)

| SEQ ID NO. | Target | Sequence |
|---|---|---|
| For the CRISPR/Cas9 system | | |
| 43 | | GGCCGCGGGAGCGCCGCGCT |
| 44 | | CGGCGGCCGGGGGACATGGC |
| 45 | | GGCCCGGCCACAGCCTGCAG |
| 46 | | TCCGAAGCGGCCCCGCTCCC |
| 47 | | GCGGAGAACAAGGGGGCCCT |
| 48 | | GCCGCCGCCGCCGCCATGTT |
| 49 | | CAGCTCCAAACCCAACATGG |
| 50 | | AGAACAAGGGGGCCCTGGGG |
| 51 | | GGGCGCTAGGCGATGAGGCG |
| 52 | | GGGCCCGCGCTCACTGCGCG |
| 53 | | ACATGGCCGGCGGGCGGAGG |
| 54 | | TCGCTGGCTCGCTGGCTGCA |
| 55 | | TCGCCGCCGCCTCCGCCCGC |
| 56 | | GCCCGAGCGCGGCGCTCCCG |
| 57 | | GGCCCCGCGCGCAGTGAGCG |
| 58 | | CTGCAGCTCCAAACCCAACA |
| 59 | | GGCCGGGGGACATGGCCGGC |
| 60 | | CGGCGACTGAGGGGGGCGCT |
| 61 | | CAGGCTGTGGCCGGGCCCGG |
| 62 | | GCCTCCCTCACCCGCGGCGG |
| 63 | | GAGGCGGCGGCGACTGAGGG |
| 64 | | GCCCACGAGAGGCAGCGCCA |
| 65 | | GCGAGCAAGCAAGCAAGCAC |
| 66 | | GGAGGCGGCGGCGACTGAGG |
| 67 | | GGGACATGGCCGGCGGGCGG |
| 68 | | CACGAGAGGCAGCGCCATGG |
| 69 | | CGCCGCCGCCATGTTGGGTT |
| 70 | | CCGTAGCTGAAGGCGCGGCC |
| 71 | | GCCCGCGCTCACTGCGCGCG |
| 72 | | GAGCGGAGCCCGCCCCGCCC |
| 73 | | CGGAGAACAAGGGGGCCCTG |
| 74 | | CCTCTCGTGGGCTCCGCTGC |
| 75 | | TTGCTTGCTTGCTCGCTCGC |
| 76 | | CGGCCGGGGGACATGGCCGG |
| 77 | | GCCCCGCTCCCTGGGCCCCG |
| 78 | | CAAACCCAACATGGCGGCGG |
| 79 | | GGCGGGGCGGGCTCCGCTCT |
| 80 | | CGAGCGAGCCCGCCGCCGCC |

(continued)

| SEQ ID NO. | Target | Sequence |
|---|---|---|
| For the CRISPR/Cas9 system | | |
| 81 | | CTCCCCGCCAGCCGCCTTCC |
| 82 | | TGTTGGGTTTGGAGCTGCAG |
| 83 | | CTCACTGCGCGCGGGGCCGC |
| 84 | | CGGCGGGCCGGGAAGGCGGC |
| 85 | | CGGGTGAGGGAGGCCGAGAG |
| 86 | | CGGGCCGGGAAGGCGGCTGG |
| 87 | | GGGCCGGGAAGGCGGCTGGC |
| 88 | | CGCGGAGAACAAGGGGGCCC |
| 89 | | GCCGTAGCTGAAGGCGCGGC |
| 90 | | GCCGCCGCCGCCGCGGGTGA |
| 91 | | CCGGCGGCGGCGGGCTCGCT |
| 92 | | CTGAAGGCGCGGCCGGGGTC |
| 93 | | GGCTGCACGGCGGGCCGGGA |
| 94 | | GCCGCGGGAGCGCCGCGCTC |
| 95 | | GCTCACTGCGCGCGGGGCCG |
| 96 | | GGGGCCCTGGGGCGGGCGAA |
| 97 | | GTGGGCTCCGCTGCAGGCTG |
| 98 | | GCGGAGCCCGCCCCGCCCCG |
| 99 | | TGGCCGGCGGGCGGAGGCGG |
| 100 | | CGGGGGACATGGCCGGCGGG |
| 101 | | CGCCATGGCGCTGCCTCTCG |
| 102 | | GAACAAGGGGGCCCTGGGGC |
| 103 | | GCTCCGCTGCAGGCTGTGGC |
| 104 | | TTGCTCGCTCGCTGGCTCGC |
| 105 | | CGTAGCTGAAGGCGCGGCCG |
| 106 | | GCTGTGGCCGGGCCCGGCGG |
| 107 | | TCGCTGGCTGCACGGCGGGC |
| 108 | | GCGGCGGCGGCCGGGGGACA |
| 109 | | GGCCGGGAAGGCGGCTGGCG |
| 110 | | GCCGCCGCCGCGGGTGAGGG |
| 111 | | TCCCTCACCCGCGGCGGCGG |
| 112 | | ACCCAACATGGCGGCGGCGG |
| 113 | | AGCGGGGCCGCTTCGGAGCC |
| 114 | | CCGCGCTCGGGCGGCGGCGG |
| 115 | | AGCGGAGCCCGCCCCGCCCC |
| 116 | | CTCCGCTGCAGGCTGTGGCC |
| 117 | | CGCTGGCTGCACGGCGGGCC |
| 118 | | GCGGCTGGCGGGGAGAGGCC |

(continued)

| SEQ ID NO. | Target | Sequence |
|---|---|---|
| For the CRISPR/Cas9 system | | |
| 119 | | TGGCCGGGCCCGGCGGCGGC |
| 120 | | GCCCCGGGGCCCAGGGAGCG |
| 121 | | CTGCAGGCTGTGGCCGGGCC |
| 122 | | CGCCGCCGCCGCCGCCATGT |
| 123 | | CGCTCGGGCGGCGGCGGCGG |
| 124 | | CCGCTCCCTGGGCCCCGGGG |
| 125 | | CTCACCCGCGGCGGCGGCGG |
| 126 | | GGCGGAGGCGGCGGCGACTG |
| 127 | | GCGCCGCGCTCGGGCGGCGG |
| 128 | | GCGGCGGCGGCGGCGGCCGG |
| 129 | | GGAAGGCGGCTGGCGGGGAG |
| 130 | | CCGCCCCGGGGCCCAGGGAG |
| 131 | | GTGGCCGGGCCCGGCGGCGG |
| 132 | | CCCGCCCCGCCCCGGGGCCC |
| 133 | | CCGCCCCGCCCCGGGGCCCA |
| 134 | | GGCCCCGCTCCCTGGGCCCC |
| 135 | | GGCGGCTGGCGGGGAGAGGC |
| 136 | | GCTCCCTGGGCCCCGGGGCG |
| 137 | | CGGAGCCGCCGCCGCCGCCG |
| 138 | | CCCTGGGCCCCGGGGCGGGG |
| 139 | | CGCCCCGGGGCCCAGGGAGC |
| 140 | | CGGCCCCGCTCCCTGGGCCC |
| 141 | | CCTGGGCCCCGGGGCGGGGC |
| 142 | | GAGCCCGCCGCCGCCGGGCC |
| 143 | | CGCTCCCTGGGCCCCGGGGC |
| 144 | | CGCCGCCGCCGCCGCGGGTG |
| 145 | | | CACTTGACTGGAATCCACCA |
| 146 | | ACACTTGACTGGAATCCACC |
| 147 | Targeting gRNA of SPPL3 exon 2 | ACCATAGACTATAAGAAGAA |
| 148 | | TCCATTCTTCTTATAGTCTA |
| 149 | | CAGAAATGTAGACACTTGAC |
| 150 | | AAGACAGTAATAGTTCTTCT |
| 151 | Targeting gRNA of SPPL3 exon 3 | AAAGACAGTAATAGTTCTTC |
| 152 | | AGTTCTTCTGGGTCTTTCAA |
| 153 | | GGACTTTGAAAATCAAGATA |

(continued)

| SEQ ID NO. | Target | Sequence |
|---|---|---|
| For the CRISPR/Cas9 system | | |
| 154 | Targeting gRNA of SPPL3 exon 4 | AGAGAGACAGATGCTCCAAT |
| 155 | | AGACAGATGCTCCAATTGGA |
| 156 | | CCTGGGTAGAGTCAATTGTT |
| 157 | | CAGGCTCTGTTCCTTCCAAT |
| 158 | | AATTGGAAGGAACAGAGCCT |
| 159 | | CCAAACAATTGACTCTACCC |
| 160 | | CAATTGGAAGGAACAGAGCC |
| 161 | Targeting gRNA of SPPL3 exon 5 | AGCAGGGTCTTGTTAAATAC |
| 162 | | CTTGTTAAATACTGGCACAT |
| 163 | | TTGTTAAATACTGGCACATC |
| 164 | | TTAAATACTGGCACATCGGG |
| 165 | Targeting gRNA of SPPL3 exon 6 | GGCCAGTGAGAACCCAGATG |
| 166 | | GAAACGTCCACAGCAACCAA |
| 167 | | TCTGGGTTCTCACTGGCCAT |
| 168 | | GTCCTCATCTGGGTTCTCAC |
| 169 | | CACTGGCCATTGGCTTCTCA |
| 170 | | TGTCATGCTCGTCCTCATCT |
| 171 | | CTGTCATGCTCGTCCTCATC |
| 172 | Targeting gRNA of SPPL3 exon 7 | AGGCAGGAGACCTTGAGGCT |
| 173 | | CAGGCGGACAAAGGCGATCA |
| 174 | | AGGCGATCATGGCGACACAG |
| 175 | | GAGGCTCGGCAGGCGGACAA |
| 176 | | CATGGCGACACAGAGGCCCA |
| 177 | | AGGAGACCTTGAGGCTCGGC |
| 178 | | TGTCCGCCTGCCGAGCCTCA |
| 179 | | TGAGAAGCCCTGAGAGAAGC |
| 180 | | GAAGCAGGCAGGAGACCTTG |
| 181 | | AGACCTTGAGGCTCGGCAGG |
| 182 | | AAGCCCTGAGAGAAGCAGGC |
| 183 | | GTCTCCTGCCTGCTTCTCTC |

(continued)

| SEQ ID NO. | Target | Sequence |
|---|---|---|
| For the CRISPR/Cas9 system | | |
| 184 | | ATCCCCTTGACGTTCTATCC |
| 185 | | GGAACATCACGCCCAACATT |
| 186 | | TCCACCTGGGGCCCAATGTT |
| 187 | | TCTATCCCGGAAGCTCCACC |
| 188 | | GCTTCCGGGATAGAACGTCA |
| 189 | | ACGTCAAGGGGATTGTCAGC |
| 190 | | AGGAACATCACGCCCAACAT |
| 191 | | TATCCCGGAAGCTCCACCTG |
| 192 | | GGTGAAGGTGGCCACTCAGC |
| 193 | | CTTCCGGGATAGAACGTCAA |
| 194 | | CTATCCCGGAAGCTCCACCT |
| 195 | | TTCCGGGATAGAACGTCAAG |
| 196 | Targeting gRNA of SPPL3 exon 8 | GGGATTGTCAGCCGGCTGAG |
| 197 | | GACGTTGCTATTGAAGATGT |
| 198 | | CAATAGCAACGTCATGGTGA |
| 199 | | TAGCAACGTCATGGTGAAGG |
| 200 | | CATCTTCAATAGCAACGTCA |
| 201 | | GCCCAACATTGGGCCCCAGG |
| 202 | | CTCCACCTGGGSGCCCAATGT |
| 203 | | GGGCCCCAGGTGGAGCTTCC |
| 204 | | CACGCCCAACATTGGGCCCC |
| 205 | | TTTCCAGGCAGAGACAGGCG |
| 206 | | TGGGCCCCAGGTGGAGCTTC |
| 207 | | CCTGTCTCTGCCTGGAAAAC |
| 208 | | GTTCCTCGCCTGTCTCTGCC |

(continued)

| SEQ ID NO. | Target | Sequence |
|---|---|---|
| For the CRISPR/Cas9 system | | |
| 209 | | ACAAAGCATAGTAGGAGACC |
| 210 | | GATGAGGGTGCAGTGAAAGT |
| 211 | | AGCGAAGGACAAAGCATAGT |
| 212 | | GATGTCTCCGATGCCCAACA |
| 213 | | ACTACAAAAAGCAAGCCAGT |
| 214 | | CTGGACCTGCCAACATCTCC |
| 215 | | GGCAGCCACTTCTCCATGTT |
| 216 | | GGCCCCACAGGAGTCCCCAC |
| 217 | | CTGCATGCGCCCGGAGATGT |
| 218 | | CATCTCCGGGCGCATGCAGA |
| 219 | | TACTTTCACTGCACCCTCAT |
| 220 | | ATCGGAGACATCGTTATGCC |
| 221 | | CGATGCCCAACATGGAGAAG |
| 222 | | AAGCCAGTGGGGACTCCTGT |
| 223 | Targeting gRNA of SPPL3 exon 9 | CTACAAAAAGCAAGCCAGTG |
| 224 | | AGCCAGTGGGGACTCCTGTG |
| 225 | | ATGCGCCCGGAGATGTTGGC |
| 226 | | TGGCAGCCACTTCTCCATGT |
| 227 | | CCTGGACCTGCCAACATCTC |
| 228 | | AACTACAAAAAGCAAGCCAG |
| 229 | | GGAGACCTTCTGCATGCGCC |
| 230 | | CAAGCCAGTGGGGACTCCTG |
| 231 | | GGAGATGTTGGCAGGTCCAG |
| 232 | | CACTTCTCCATGTTGGGCAT |
| 233 | | CGGAGATGTTGGCAGGTCCA |
| 234 | | GGCAGGTCCAGGGGCCCCAC |
| 235 | | CCGGAGATGTTGGCAGGTCC |
| 236 | | GGGGACTCCTGTGGGGCCCC |
| 237 | | ACAAAGCATAGTAGGAGACC |

(continued)

| SEQ ID NO. | Target | Sequence |
|---|---|---|
| For the CRISPR/Cas9 system | | |
| 238 | Targeting gRNA of SPPL3 exon 10 | AAATAGGCCATCGTGAGGAG |
| 239 | | AGGAGTGGCAATAAAGTAAA |
| 240 | | TTTATTGCCACTCCTCACGA |
| 241 | | TGGCACCAAATAGAGAAGGG |
| 242 | | AAATGGCACCAAATAGAGAA |
| 243 | | GGCACCAAATAGAGAAGGGC |
| 244 | | TAAATGGCACCAAATAGAGA |
| 245 | | CCAGCCCGCCCTTCTCTATT |
| 246 | | CCAAATAGAGAAGGGCGGGC |
| 247 | | ATAGAGAAGGGCGGGCTGGG |
| 248 | | CAAATAGAGAAGGGCGGGCT |
| 249 | | GAAGGGCGGGCTGGGCGGCC |
| 250 | Targeting gRNA of SPPL3 exon 11 | AAGGCTCAGACCACATCCGC |
| 251 | | GCTCAGACCACATCCGCCGG |
| 252 | | GTCCAGCAGCTCCCGATTCC |
| 253 | | CTGCTGGACTTGGAGTGGAA |
| 254 | | TTCCAGGAATCGGGAGCTGC |
| 255 | | GGGAGCTGCTGGACTTGGAG |
| 256 | | GAATCGGGAGCTGCTGGACT |
| For CRISPR/Cas12 system | | |
| 257 | Targeting sgRNA of TRAC | CAGGAGGAGGATTCGGAACC |
| 258 | | TCGACCAGCTTGACATCACA |
| 259 | | AATCTGCTCATGACGCTGCG |
| 260 | | TGGATATCTGTGGGACAAGA |
| 261 | Targeting sgRNA of SPPL3 exon 2 | TTCTTATAGTCTATGGTAGT |
| 262 | | CCTGGTGGATTCCAGTCAAG |
| 263 | | CTGATTTCCATTCTTCTTAT |
| 264 | | TGATTTCCATTCTTCTTATA |
| 265 | | CCATTCTTCTTATAGTCTAT |
| 266 | | CATTCTTCTTATAGTCTATG |
| 267 | | CCTGAAACTACCATAGACTA |
| 268 | | TTATAGTCTATGGTAGTTTC |

(continued)

| For CRISPR/Cas12 system | | |
|---|---|---|
| 269 | | TGGGTCTTTCAATGGCAACA |
| 270 | | CAATGGCAACAGCACCAATA |
| 271 | | GAAAATCAAGATAAGGAGAA |
| 272 | | AAAATCAAGATAAGGAGAAA |
| 273 | | TTCTGGGTCTTTCAATGGCA |
| 274 | Targeting sgRNA of SPPL3 exon 3 | AATGGCAACAGCACCAATAA |
| 275 | | CTGTCTTTCTCCTTATCTTG |
| 276 | | AAAGACCCAGAAGAACTATT |
| 277 | | CCATTGAAAGACCCAGAAGA |
| 278 | | GTGCTGTTGCCATTGAAAGA |
| 279 | | TTGGTGCTGTTGCCATTGAA |
| 280 | | CTCCTTATCTTGATTTTCAA |
| 281 | | CAGGCATCCAAACAATTGAC |
| 282 | | CAATTGGAGCATCTGTCTCT |
| 283 | | TAGTAATGTTCTTCTTCTTT |
| 284 | | TTCTTTGACTCAGTTCAAGT |
| 285 | | TTTGACTCAGTTCAAGTAGT |
| 286 | Targeting sgRNA of SPPL3 exon 4 | GACTCAGTTCAAGTAGTTTT |
| 287 | | CTTCCAATTGGAGCATCTGT |
| 288 | | TTCTTCTTTGACTCAGTTCA |
| 289 | | AAGTAGTTTTTACAATATGT |
| 290 | | TGAGGTGAGCAGGGTCTTGT |
| 291 | | TGCTTTTCTTCTCCTCCCGA |
| 292 | | TCTTCTCCTCCCGATGTGCC |
| 293 | | TCCTCCCGATGTGCCAGTAT |
| 294 | Targeting sgRNA of SPPL3 exon 5 | TTGCAACGATAGCTTTTGCT |
| 295 | Targeting sgRNA of SPPL3 exon 6 | GGTTGCTGTGGACGTTTCAC |
| 296 | | TCACTGGCCATTGGCTTCTC |
| 297 | | GTCCGCCTGCCGAGCCTCAA |
| 298 | Targeting sgRNA of SPPL3 exon 7 | TCTCAGGGCTTCTCATCTAT |
| 299 | | TCATCTATGATGTCTTTTGG |
| 300 | | AATAGCAACGTCATGGTGAA |
| 301 | | ACCATGACGTTGCTATTGAA |
| 302 | | CGGGATAGAACGTCAAGGGG |
| 303 | | TATCCCGGAAGCTCCACCTG |
| 304 | Targeting sgRNA of SPPL3 exon 8 | CTCGCCTGTCTCTGCCTGGA |

(continued)

| For CRISPR/Cas12 system | | |
|---|---|---|
| 305 | | TCCATGTTGGGCATCGGAGA |
| 306 | | GTCCTTCGCTATGACAACTA |
| 307 | | GCTATGACAACTACAAAAAG |
| 308 | Targeting sgRNA of SPPL3 exon 9 | TTGTAGTTGTCATAGCGAAG |
| 309 | | TGCATGCGCCCGGAGATGTT |
| 310 | | CACTGCACCCTCATCGGATA |
| 311 | | TCTTACCTACAAAGTATCCG |
| 312 | | TCTATTTGGTGCCATTTACT |
| 313 | Targeting sgRNA of SPPL3 exon 10 | ATTGCCACTCCTCACGATGG |
| 314 | | AAATAGGCCATCGTGAGGAG |
| 315 | Targeting sgRNA of SPPL3 exon 11 | CCACTCCAAGTCCAGCAGCT |
| 316 | | CAGGAATCGGGAGCTGCTGG |

**Claims**

1. An engineered T cell, wherein the T cell is modified to eliminate or reduce the expression and/or function of an endogenous T cell receptor (TCR) protein or a functional fragment thereof and/or an SPPL3 protein or a functional fragment thereof.

2. The engineered T cell according to claim 1, wherein the expression and/or function of an $\alpha$ subunit (TCR$\alpha$) and/or a $\beta$ subunit (TCR$\beta$) of the endogenous T cell receptor (TCR) is eliminated or reduced after modification.

3. The engineered T cells according to claim 1 or 2, further, wherein the expression and/or function of $\beta$-2 microglobulin (B2M) is retained, or eliminated or reduced.

4. The engineered T cell according to any one of claims 1 to 3, further comprising or expressing an engineered receptor.

5. The engineered T cell according to claim 4, wherein the engineered receptor is any one or more selected from the group consisting of: a chimeric antigen receptor (CAR), an engineered TCR, and a T cell antigen conjugate (TAC).

6. A method for preparing the engineered T cell according to any one of claims 1 to 5, wherein the modification comprises eliminating or reducing the expression and/or function of the TCR protein or a functional fragment thereof and/or the SPPL3 protein or a functional fragment thereof by any one or more selected from the group consisting of: disruption or knockout of coding genes, frameshift mutation or knockout, inhibition of coding gene transcription, disruption or clearance of mRNA, inhibition of coding gene expression and inhibition of the proteins.

7. The method according to claim 6, wherein the modification eliminates or reduces the expression of TCR or a functional fragment thereof and SPPL3 or a functional fragment thereof by RNA interference (RNAi), optionally, the RNAi silences or inhibits the expression of TCR or a functional fragment thereof and SPPL3 or a functional fragment thereof by small interfering RNA (siRNA), short hairpin RNA (shRNA) or small RNA (miRNA).

8. The method according to claim 6, wherein the modification comprises eliminating or reducing the expression of TCR or a functional fragment thereof and SPPL3 or a functional fragment thereof via a CRISPR/Cas system-mediated gene editing or RNA editing method, the gene editing or RNA editing method uses a guide RNA (gRNA) targeting SPPL3 and/or TRAC, and the gRNA uses a guide sequence complementary to the SPPL3 genomic region at positions 120,903,845 to 120,904,358, 120,810,809 to 120,810,886, 120,791,469 to 120,791,557, 120,784,474 to 120,784,593, 120,783,674 to 120,783,752, 120,782,655 to 120,782,767, 120,768,953 to 120,769,059, 120,768,325 to 120,768,488, 120,767,394 to 120,767,593, 120,766,263 to 120,766,372, or 120,764,999 to 120,765,070 of human chromosome 12; and/or uses a guide sequence complementary to the TRAC genomic region at positions 23,016,448 to 23,016,490 of human chromosome 14.

9. The method according to claim 8, wherein:

the guide sequence complementary to the SPPL3 genomic region is any one or more selected from the group consisting of: SEQ ID NO:68, SEQ ID NO:145, SEQ ID NO:150, SEQ ID NO:154, SEQ ID NO:155, SEQ ID NO:159, SEQ ID NO:161, SEQ ID NO:165, SEQ ID NO:172, SEQ ID NO:176, SEQ ID NO:196, SEQ ID NO:203, SEQ ID NO:204, SEQ ID NO: 209, SEQ ID NO:212, SEQ ID NO:241, SEQ ID NO:250, SEQ ID NO:261, SEQ ID NO:269, SEQ ID NO:270, SEQ ID NO:271, SEQ ID NO:272, SEQ ID NO:273, SEQ ID NO:274, SEQ ID NO:275, SEQ ID NO:276, SEQ ID NO:277, SEQ ID NO:278, SEQ ID NO:279, SEQ ID NO:280, SEQ ID NO:281, SEQ ID NO:282, SEQ ID NO:283, SEQ ID NO:284, SEQ ID NO:285, SEQ ID NO:286, SEQ ID NO:287, SEQ ID NO:288, SEQ ID NO:289, SEQ ID NO:290, SEQ ID NO:291, SEQ ID NO:292, SEQ ID NO:293, SEQ ID NO:294, SEQ ID NO:295, SEQ ID NO:297, SEQ ID NO:300, SEQ ID NO:305, SEQ ID NO:312 and SEQ ID NO:315 and/or the guide sequence complementary to the TRAC genome region is any one or more selected from the group consisting of: SEQ ID NO:1, SEQ ID NO:2, SEQ ID NO:257, SEQ ID NO:258, SEQ ID NO:259 and SEQ ID NO:260.

10. The method according to claim 8, wherein the gRNA uses a guide sequence complementary to the SPPL3 genomic region at positions 120,791,469 to 120,791,557, 120,783,674 to 120,783,752, or 120,784,474 to 120,784,593 of human chromosome 12.

11. The method according to claim 10, wherein:
the guide sequence complementary to the SPPL3 genomic region is any one or more selected from the group consisting of:
SEQ ID NO:150, SEQ ID NO:154, SEQ ID NO:155, SEQ ID NO:159, SEQ ID NO:161, SEQ ID NO:269, SEQ ID NO:270, SEQ ID NO:271, SEQ ID NO:272, SEQ ID NO:273, SEQ ID NO:274, SEQ ID NO:275, SEQ ID NO:276, SEQ ID NO:277, SEQ ID NO:278, SEQ ID NO:279, SEQ ID NO:280, SEQ ID NO:281, SEQ ID NO:282, SEQ ID NO:283, SEQ ID NO:284, SEQ ID NO:285, SEQ ID NO:286, SEQ ID NO:287, SEQ ID NO:288, SEQ ID NO:289, SEQ ID NO:290, SEQ ID NO:291, SEQ ID NO:292, SEQ ID NO:293 and SEQ ID NO:294.

12. The method according to any one of claims 8 to 11, wherein the gRNA is chemically modified.

13. The method according to claim 12, wherein the chemical modification comprises 2'-O-methylation on the ribose of the nucleotide or 3' thiophosphate bond modification between nucleotides or both.

14. The method according to claim 13, wherein the modification is 2'-O-methylation modification on the ribose of the first three nucleotides at the 5' end, 2'-O-methylation modification on the ribose of the last three nucleotides at the 3' end, internucleotide 3' thiophosphorylation modification on the first three nucleotides at the 5' end, or internucleotide 3' thiophosphorylation modification on the last three nucleotides at the 3' end.

15. The method according to any one of claims 8 to 14, wherein the CRISPR/Cas system is a CRISPR/Cas9 system or a CRISPR/Cas12 system.

16. A method for prolonging the in vivo half-life of engineered cells, comprising modifying the engineered cells to eliminate or reduce the expression and/or function of an SPPL3 protein or a functional fragment thereof.

17. The method according to claim 16, the modification eliminates or reduces the expression and/or function of SPPL3 protein or a functional fragment thereof by any one or more selected from the group consisting of: disruption of the coding gene, frameshift mutation or knockout, inhibition of coding gene transcription, disruption or clearance of mRNA, inhibition of coding gene expression and inhibition of the protein, optionally, the modification eliminates or reduces the expression of SPPL3 or a functional fragment thereof by RNA interference (RNAi), for example, the RNAi silences or inhibits the expression of SPPL3 protein or a functional fragment thereof by small interfering RNA (siRNA), short hairpin RNA (shRNA) or small RNA (miRNA).

18. The method according to claim 16, wherein the modification eliminates or reduces the expression of SPPL3 protein or a functional fragment thereof via a CRISPR/Cas system-mediated gene editing or RNA editing method, wherein the gene editing or RNA editing method uses a guide RNA (gRNA) targeting SPPL3, for example, the gRNA uses a guide sequence complementary to the SPPL3 genomic region at positions 120,903,845 to 120,904,358, 120,810,809 to 120,810,886, 120,791,469 to 120,791,557, 120,784,474 to 120,784,593, 120,783,674 to 120,783,752, 120,782,655 to 120,782,767, 120,768,953 to 120,769,059, 120,768,325 to 120,768,488, 120,767,394 to 120,767,593, 120,766,263 to 120,766,372, or 120,764,999 to 120,765,070 of human chromosome 12 to knock out the SPPL3 coding gene or cause a frameshift mutation in the SPPL3 coding gene.

19. The method according to claim 16, wherein the gRNA uses a guide sequence complementary to the SPPL3 genomic region at positions 120,791,469 to 120,791,557, 120,783,674 to 120,783,752, or 120,784,474 to 120,784,593of human chromosome 12.

20. The method according to any one of claims 16 to 19, wherein the engineered cell has or expresses normal levels of MHC-I protein.

21. The method according to any one of claims 16 to 20, wherein the engineered cell is an immune cell or a precursor cell thereof.

22. The method according to claim 21, wherein the immune cell is any one or more selected from the group consisting of: T cells, B cells, natural killer (NK) cells, macrophages, and DC cells.

23. The method according to claim 22, wherein the immune cell is an αβ T cell, and the expression and/or function of endogenous TCRα and/or TCRβ or a functional fragment thereof of the αβ T cell is eliminated or reduced.

24. The method according to any one of claims 16 to 23, wherein the engineered cell further comprises or expresses an engineered receptor.

25. The method according to claim 24, wherein the engineered receptor is any one or more selected from the group consisting of: a chimeric antigen receptor (CAR), an engineered TCR, and a T cell antigen coupler (TAC).

26. A guide RNA (gRNA) for eliminating or reducing the expression of an SPPL3 protein or a functional fragment thereof via CRISPR/Cas system-mediated gene editing methods, comprising any one nucleotide sequence selected from the group consisting of:
SEQ ID NO:68, SEQ ID NO:145, SEQ ID NO:150, SEQ ID NO:154, SEQ ID NO:155, SEQ ID NO:159, SEQ ID NO:161, SEQ ID NO:165, SEQ ID NO:172, SEQ ID NO:176, SEQ ID NO:196, SEQ ID NO:203, SEQ ID NO:204, SEQ ID NO:209, SEQ ID NO:212, SEQ ID NO:241, SEQ ID NO:250, SEQ ID NO:261, SEQ ID NO:269, SEQ ID NO:270, SEQ ID NO:271, SEQ ID NO:272, SEQ ID NO:273, SEQ ID NO:274, SEQ ID NO:275, SEQ ID NO:276, SEQ ID NO:277, SEQ ID NO:278, SEQ ID NO:279, SEQ ID NO:280, SEQ ID NO:281, SEQ ID NO:282, SEQ ID NO:283, SEQ ID NO:284, SEQ ID NO:285, SEQ ID NO:286, SEQ ID NO:287, SEQ ID NO:288, SEQ ID NO:289, SEQ ID NO:290, SEQ ID NO:291, SEQ ID NO:292, SEQ ID NO:293, SEQ ID NO:294, SEQ ID NO:295, SEQ ID NO:297, SEQ ID NO:300, SEQ ID NO:305, SEQ ID NO:312 and SEQ ID NO:315.

27. A composition, comprising the gRNA according to claim 26.

28. A composition comprising a gRNA targeting a gene encoding a T cell receptor (TCR) or a functional fragment thereof and/or a gRNA targeting a gene encoding an SPPL3 protein or a functional fragment thereof.

29. The composition according to claim 28, wherein the gRNA targeting SPPL3 comprises a guide sequence complementary to the SPPL3 genomic region at positions 120903845 to 120904358, 120810809 to 120810886, 120791469 to 120791557, 120784474 to 120784593, 120783674 to 120783752, 120782655 to 120782767, 120768953 to 120769059, 120768325 to 120768488, 120767394 to 120767593, 120766263 to 120766372, or 120764999 to 120765070 of human chromosome 12.

30. The composition according to claim 29, wherein the gRNA targeting SPPL3 comprises any one guide sequence selected from the group consisting of:
SEQ ID NO:68, SEQ ID NO:145, SEQ ID NO:150, SEQ ID NO:154, SEQ ID NO:155, SEQ ID NO:159, SEQ ID NO:161, SEQ ID NO:165, SEQ ID NO:172, SEQ ID NO:176, SEQ ID NO:196, SEQ ID NO:203, SEQ ID NO:204, SEQ ID NO:209, SEQ ID NO:212, SEQ ID NO:241, SEQ ID NO:250, SEQ ID NO:261, SEQ ID NO:269, SEQ ID NO:270, SEQ ID NO:271, SEQ ID NO:272, SEQ ID NO:273, SEQ ID NO:274, SEQ ID NO:275, SEQ ID NO:276, SEQ ID NO:277, SEQ ID NO:278, SEQ ID NO:279, SEQ ID NO:280, SEQ ID NO:281, SEQ ID NO:282, SEQ ID NO:283, SEQ ID NO:284, SEQ ID NO:285, SEQ ID NO:286, SEQ ID NO:287, SEQ ID NO:288, SEQ ID NO:289, SEQ ID NO:290, SEQ ID NO:291, SEQ ID NO:292, SEQ ID NO:293, SEQ ID NO:294, SEQ ID NO:295, SEQ ID NO:297, SEQ ID NO:300, SEQ ID NO:305, SEQ ID NO:312, and SEQ ID NO:315.

31. The composition according to claim 29, wherein the gRNA uses a guide sequence complementary to the SPPL3 genomic region at positions 120791469 to 120791557, 120783674 to 120783752, or 120784474 to 120784593 of human chromosome 12.

32. The composition according to claim 31, wherein:
    the guide sequence complementary to the SPPL3 genomic region is any one or more selected from the group consisting of:
    SEQ ID NO:150, SEQ ID NO:154, SEQ ID NO:155, SEQ ID NO:159, SEQ ID NO:161, SEQ ID NO:269, SEQ ID NO:270, SEQ ID NO:271, SEQ ID NO:272, SEQ ID NO:273, SEQ ID NO:274, SEQ ID NO:275, SEQ ID NO:276, SEQ ID NO:277, SEQ ID NO:278, SEQ ID NO:279, SEQ ID NO:280, SEQ ID NO:281, SEQ ID NO:282, SEQ ID NO:283, SEQ ID NO:284, SEQ ID NO:285, SEQ ID NO:286, SEQ ID NO:287, SEQ ID NO:288, SEQ ID NO:289, SEQ ID NO:290, SEQ ID NO:291, SEQ ID NO:292, SEQ ID NO:293, and SEQ ID NO:294.

33. The composition according to any one of claims 28 to 32, wherein the T cell receptor gene is the TRAC gene.

34. The composition according to claim 33, wherein the gRNA targeting the T cell receptor gene comprises a guide sequence complementary to the TRAC genomic region at positions 23016448 to 23016490 of human chromosome 14.

35. The composition according to claim 34, wherein the guide sequence comprises a nucleotide sequence as set forth in SEQ ID NO:1, SEQ ID NO:2, SEQ ID NO:257, SEQ ID NO:258, SEQ ID NO:259, or SEQ ID NO:260.

Figure 1A

Figure 1B

Figure 2A

Figure 2B

CAR+ % of U-CART Cells on Day 9

Figure 2C

Viability of U-CART Cells on Day 9

Figure 2D

Viability of U-CART Cells on Day 9

Figure 2E

Cell Proliferation

Figure 3A

Figure 3B

TCR Knockout Efficiency % of U-CART Cells on Day 9

Figure 4A

Figure 4B

Figure 4C

Viability of U-CART Cells on Day 9

Figure 4D

Cell Number of U-CART Cells on Day 9

Figure 4E

UCART-Gene Knockout Efficiency %

Figure 5A

UCART Viability %

Figure 5B

Proliferation

Figure 6A

Viability

Figure 6B

UCART Viability %

Figure 6C

UCART-TCR Knockout Efficiency %

Figure 7A

UCART Viability %

Figure 7B

Proliferation

Figure 8A

Viability

Figure 8B

Figure 8C

Figure 9

Figure 10

Figure 11A

Figure 11B

**FasL**

Figure 12

Figure 13

Figure 14

Figure 15

|  | G1:Tumor-Bearing Control Group | G2:T Cell Control Group | G3: CAR-T | G6-1: UCART |

Day 8 Post Administration

Day 15 Post Administration

Day 28 Post Administration

Day 39 Post Administration

Min:100000
Max:2000000

Figure 16

Mean Fluorescence Intensity

- ▲ G1:Tumor-Bearing Control Group
- ● G2:T Cell Control Group
- ■ G3: CAR-T
- ◆ G6-1: UCART

Residual fluorescence of Tumor Cells

Detection Time after In Vivo UCART Injection

Figure 17

## INTERNATIONAL SEARCH REPORT

| International application No. |
|---|
| **PCT/CN2024/113033** |

| A. | CLASSIFICATION OF SUBJECT MATTER |
|---|---|

C12N5/10(2006.01)i;  C12N15/113(2010.01)i

According to International Patent Classification (IPC) or to both national classification and IPC

| B. | FIELDS SEARCHED |
|---|---|

Minimum documentation searched (classification system followed by classification symbols)

IPC:  C12N

Documentation searched other than minimum documentation to the extent that such documents are included in the fields searched

Electronic data base consulted during the international search (name of data base and, where practicable, search terms used)

CNKI, CNTXT, DWPI, ENTXT, ELSEVIER, ISI Web of Knowledge, 中国专利生物序列检索系统, China Patents Biological Sequence Search System, Genbank, EMBL, STN, PubMed: 嵌合抗原受体, 免疫细胞, NK细胞, 自然杀伤细胞, Natural Killer cell, 相关序列, T细胞, T细胞受体, TCR, SPPL3, 信号肽肽酶样3, β-2微球蛋白, B2M, RNA干扰, RNAi, 半衰期, 通用型 CAR-T, U-CART, 移植物抗宿主病, GvHD, 激活诱导的免疫效应细胞死亡, AICD, Activation Induced Cell Death, 宿主抗移 植物效应, HVG, IMP2, PSH1, PSL4, 向导RNA, gRNA, CRISPR/Cas, sgRNA, 基因敲除, knockout, chimeric antigen receptor, CAR-T, immune cell, T cell, half-life

| C. | DOCUMENTS CONSIDERED TO BE RELEVANT |
|---|---|

| Category* | Citation of document, with indication, where appropriate, of the relevant passages | Relevant to claim No. |
|---|---|---|
| X | CN 114901813 A (EDIGENE (BEIJING) BIOTECHNOLOGY CO., LTD.) 12 August 2022 (2022-08-12)<br>claims 1-3, 8-14, and 24, specific embodiments, and table 1 | 1-9, 12-15, 28, 33-35 |
| X | US 2022127591 A1 (UNIVERSITY OF COPENHAGEN et al.) 28 April 2022 (2022-04-28)<br>table 5 | 28 |
| X | CN 114867850 A (EDIGENE (BEIJING) BIOTECHNOLOGY CO., LTD.) 05 August 2022 (2022-08-05)<br>claims 1-22 | 1-8, 12-15, 28, 33-34 |
| A | US 2023302134 A1 (YALE UNIVERSITY) 28 September 2023 (2023-09-28)<br>entire document | 1-35 |

☑ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **11 November 2024** | **15 November 2024** |

| Name and mailing address of the ISA/CN | Authorized officer |
|---|---|
| **China National Intellectual Property Administration (ISA/ CN)**<br>**China No. 6, Xitucheng Road, Jimenqiao, Haidian District, Beijing 100088** | |
| | Telephone No. |

Form PCT/ISA/210 (second sheet) (July 2022)

**EP 4 763 979 A1**

**INTERNATIONAL SEARCH REPORT**

International application No.

**PCT/CN2024/113033**

**C.    DOCUMENTS CONSIDERED TO BE RELEVANT**

| Category* | Citation of document, with indication, where appropriate, of the relevant passages | Relevant to claim No. |
|---|---|---|
| A | CN 107354156 A (THE FIFTH AFFILIATED HOSPITAL OF GUANGZHOU MEDICAL UNIVERSITY) 17 November 2017 (2017-11-17) <br> entire document | 1-35 |
| A | US 2021047405 A1 (NOVARTIS AG et al.) 18 February 2021 (2021-02-18) <br> entire document | 1-35 |
| A | CN 107236741 A (THE FIFTH AFFILIATED HOSPITAL OF GUANGZHOU MEDICAL UNIVERSITY) 10 October 2017 (2017-10-10) <br> entire document | 1-35 |

Form PCT/ISA/210 (second sheet) (July 2022)

58

| **INTERNATIONAL SEARCH REPORT** | International application No. |
| --- | --- |
| | **PCT/CN2024/113033** |

| **Box No. I** **Nucleotide and/or amino acid sequence(s) (Continuation of item 1.c of the first sheet)** |
| --- |

1.  With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

          ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

**INTERNATIONAL SEARCH REPORT**
Information on patent family members

International application No.

**PCT/CN2024/113033**

| Patent document cited in search report | | | Publication date (day/month/year) | Patent family member(s) | | | Publication date (day/month/year) |
|---|---|---|---|---|---|---|---|
| CN | 114901813 | A | 12 August 2022 | US | 2023059884 | A1 | 23 February 2023 |
| | | | | EP | 4086340 | A1 | 09 November 2022 |
| | | | | WO | 2021136176 | A1 | 08 July 2021 |
| | | | | JP | 2023509058 | A | 06 March 2023 |
| US | 2022127591 | A1 | 28 April 2022 | WO | 2020047282 | A1 | 05 March 2020 |
| | | | | US | 2023416715 | A1 | 28 December 2023 |
| | | | | US | 11708569 | B2 | 25 July 2023 |
| CN | 114867850 | A | 05 August 2022 | WO | 2021136415 | A1 | 08 July 2021 |
| | | | | JP | 2023516538 | A | 20 April 2023 |
| | | | | EP | 4086341 | A1 | 09 November 2022 |
| | | | | US | 2023054266 | A1 | 23 February 2023 |
| US | 2023302134 | A1 | 28 September 2023 | JP | 2023538303 | A | 07 September 2023 |
| | | | | KR | 20230044537 | A | 04 April 2023 |
| | | | | WO | 2022036180 | A1 | 17 February 2022 |
| | | | | AU | 2021325947 | A1 | 02 March 2023 |
| | | | | CA | 3188988 | A1 | 17 February 2022 |
| | | | | EP | 4196579 | A1 | 21 June 2023 |
| CN | 107354156 | A | 17 November 2017 | | None | | |
| US | 2021047405 | A1 | 18 February 2021 | EP | 3784351 | A1 | 03 March 2021 |
| | | | | US | 2024076372 | A1 | 07 March 2024 |
| | | | | WO | 2019210153 | A1 | 31 October 2019 |
| CN | 107236741 | A | 10 October 2017 | | None | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020074001 A
- US 20140287509 A

- US 8697359 B


**Non-patent literature cited in the description**

- Overview of principles of hybridization and the strategy of nucleic acid probe assay. **TIJSSEN**. Laboratory Techniques In Biochemistry And Molecular Biology - Hybridization With Nucleic Acid Probes Part 1. Elsevier, 1993
- **HELSEN et al.** *Nat Commun.*, 2018, vol. 9 (1), 3049
- **GUILLERMO AQUINO-JARQUIN**. Novel Engineered Programmable Systems for ADAR-Mediated RNA Editing. *Mol Ther Nucleic Acids*, 2020, vol. 19, 1065-1072
- **QU et al.** *Nat Biotechnol.*, 2019, vol. 37 (9), 1059-1069
- **COX et al.** RNA editing with CRISPR-Cas13. *Science*, 2017, vol. 358 (6366), 1019-1027
- **MERKLE et al.** Precise RNA editing by recruiting endogenous ADARs with antisense oligonucleotides. *Methods Mol Biol*, 2021, vol. 2181, 331-349

- **RAUCH et al.** Programmable RNA-Guided RNA Effector Proteins Built from Human Parts. *Cell*, 2019, vol. 178 (1), 122-134.e12
- **ABUDAYYEH et al.** A cytosine deaminase for programmable single-base RNA editing. *Science*, 2019, vol. 365 (6451), 382-386
- **P. REAUTSCHNIG et al.** CLUSTER guide RNAs enable precise and efficient RNA editing with endogenous ADAR enzymes in vivo. *Nat Biotechnol.*, May 2022, vol. 40 (5), 759-768
- **NIDHI et al.** Novel CRISPR-Cas Systems: An Updated Review of the Current Achievements, Applications, and Future Research Perspectives. *Int J Mol Sci*, 2021, vol. 22 (7), 3327
- **UI-TEI et al.** *FEBS Letters*, 2000, vol. 479, 79-82
- **NISHIMASU H et al.** Crystal structure of Cas9 in complex with guide RNA and target DNA. *Cell*, 2014, vol. 156, 935-949